(19) Europäisches Patentamt
European Patent Office
Office européen des brevets

(11) **EP 4 659 564 A1**

(12) **EUROPEAN PATENT APPLICATION**
published in accordance with Art. 153(4) EPC

(43) Date of publication:
**10.12.2025 Bulletin 2025/50**

(21) Application number: **24749942.9**

(22) Date of filing: **16.01.2024**

(51) International Patent Classification (IPC):
**A01G 7/00** (2006.01)  **G01N 21/31** (2006.01)

(52) Cooperative Patent Classification (CPC):
**A01G 7/00; G01N 21/31**

(86) International application number:
**PCT/JP2024/001028**

(87) International publication number:
**WO 2024/161988 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.01.2023 JP 2023013278**

(71) Applicant: **Hiroshima University
Higashihiroshima-shi
Hiroshima 739-8511 (JP)**

(72) Inventor: **TOMINAGA Jun
Higashihiroshima-shi, Hiroshima 739-8511 (JP)**

(74) Representative: **Abel & Imray LLP
Westpoint Building
James Street West
Bath BA1 2DA (GB)**

(54) **BIOLOGICAL INFORMATION MEASURING INSTRUMENT AND BIOLOGICAL INFORMATION MEASURING METHOD**

(57) This biological information measuring instrument is characterized by comprising: a receptacle for covering at least a section of a plant leaf; and a sensor for measuring the concentration of water vapor and/or carbon dioxide on the inside and the outside of the receptacle. At least a section of the walls that constitute the receptacle is characterized by being formed of a permeable film having permeation resistance with respect to water vapor and carbon dioxide.

[FIG. 5]

EP 4 659 564 A1

**Description**

TECHNICAL FIELD

The present disclosure relates to a biological information measuring device and a method for measuring biological information, and more particularly to a biological information measuring device for plants and a method for measuring biological information for plants.

BACKGROUND ART

In order to optimize the cultivation environment and automate cultivation management, it is necessary to accurately evaluate the biological information of plants. Conventionally, the evaluation of biological information of plants has been based on the external appearance of the plant, and also on the intuition and experience of the grower.

That is, the evaluation of the biological information of plants has been performed by human visual inspection, etc., but it has been difficult for producers other than experienced producers to accurately evaluate the biological information of plants. In addition, scientific data on the biological information of plants cannot be obtained non-destructively, accurately, and easily, making it difficult to optimize the cultivation environment and automate cultivation management. Furthermore, it is preferable to detect signs of biological information before changes appear in the appearance of the plant. Therefore, a method of using a measuring device equipped with a sensor capable of acquiring gas concentration has been proposed as a method of evaluating the biological information of plants.

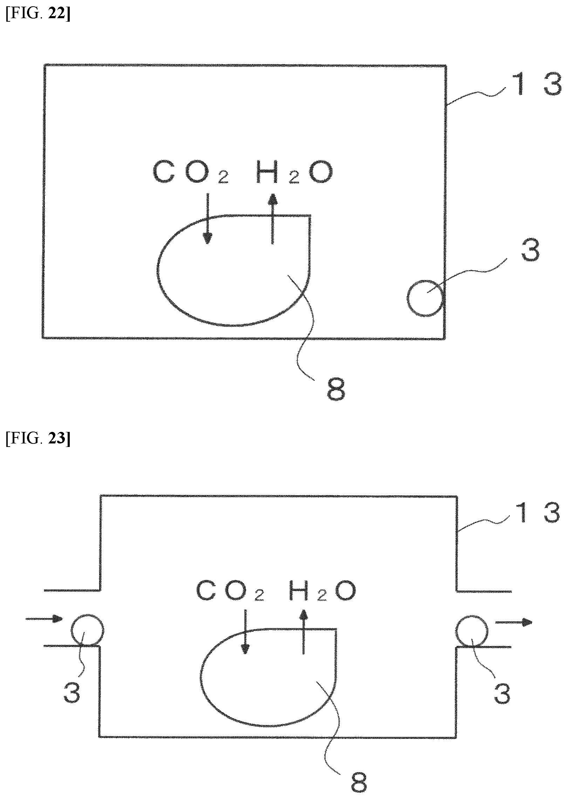

For example, a method has been proposed for measuring the transpiration rate and the carbon dioxide assimilation rate using a sealed chamber (13) equipped with a sensor (3) as shown in FIG. 22. In this measurement method, after all or part of the plant body is housed in a sealed chamber, the concentrations of water vapor ($H_2O$) and carbon dioxide ($CO_2$) in the air in the chamber and the concentrations of water vapor ($H_2O$) and carbon dioxide ($CO_2$) in the air in the chamber after a certain time has passed are obtained by the sensor, and the transpiration rate (E) and the carbon dioxide assimilation rate (A) are calculated from the difference between these concentrations ($\Delta H_2O$, $\Delta CO_2$) and the amount of air in the chamber as expressed by the following formulas (1) and (2).

[Mathematical Formula 1]

$$\text{Transpiration rate (E)} = \frac{\Delta H_2O \times \text{amount of air in the chamber}}{\text{amount of plant body}} \qquad (1)$$

$$\text{Carbon dioxide assimilation rate (A)} = \frac{\Delta CO_2 \times \text{amount of air in the chamber}}{\text{amount of plant body}} \qquad (2)$$

Also, a method has been proposed for measuring the transpiration rate (E) and the carbon dioxide assimilation rate (A) using a ventilated chamber (13) equipped with sensors (3) at the inlet and outlet, as shown in FIG. 23. In this measurement method, all or part of the plant body is housed in the ventilated chamber (13), the concentrations of water vapor ($H_2O$) and carbon dioxide ($CO_2$) at the inlet of the chamber (13) and the concentrations of water vapor ($H_2O$) and carbon dioxide ($CO_2$) at the outlet of the chamber (13) are obtained by each sensor (3), and the transpiration rate (E) and the carbon dioxide assimilation rate (A) are calculated from the difference in concentration between the inlet and outlet ($\Delta H_2O$, $\Delta CO_2$) and the flow rate of air supplied to the chamber (13) as expressed by the following formulas (3) and (4).

[Mathematical Formula 2]

$$\text{Transpiration rate (E)} = \frac{\Delta H_2O \times \text{flow rate of air in the chamber}}{\text{amount of plant body}} \qquad (3)$$

$$\text{Carbon dioxide assimilation rate (A)} = \frac{\Delta CO_2 \times \text{flow rate of air in the chamber}}{\text{amount of plant body}} \qquad (4)$$

For example, Patent Literature 1 is an example of measuring biological information of a plant using a ventilated chamber. Patent Literature 1 discloses a system for measuring the photosynthetic rate (carbon dioxide assimilation rate) of a plant, the system comprising a covering part for covering a target plant, a housing, and a sensor for measuring carbon dioxide concentration, an air stirring part for stirring the flow of air flowing into the housing is provided inside the housing, and the sensor is provided on the air inlet side and the air outlet side in the housing. Patent Literature 1 discloses that the photosynthetic rate (carbon dioxide assimilation rate) is calculated by the exhaust fan exhaust volume $\times$ [(CO$_2$ concentration of air flowing into the covering part) - (CO$_2$ concentration of air exhausted from the covering part)].

CITATION LIST

PATENT LITERATURE

[Patent Literature 1] Japanese Laid-Open Patent Publication No. 2019-170247

SUMMARY OF INVENTION

TECHNICAL PROBLEM

As described above, conventional measurement methods have been disclosed that use a sealed chamber as shown in FIG. 22 and a ventilated chamber as shown in FIG. 23 and in Patent Literature 1 to measure plant biological information such as the transpiration rate (E) or the carbon dioxide assimilation rate (A).

However, in the measurement method using a sealed chamber, it is necessary to seal all or part of the plant body in the chamber. Therefore, as time passes, the amount of water vapor in the air inside the chamber increases due to the transpiration of the plant body, and the amount of carbon dioxide in the air inside the chamber decreases due to the photosynthesis of the plant body. Eventually, the water vapor in the air inside the chamber becomes saturated, while the carbon dioxide is depleted, so that it becomes impossible to measure the transpiration rate (E) and the carbon dioxide assimilation rate (A) of the plant body. Therefore, in order to continuously measure the transpiration rate (E) or the carbon dioxide assimilation rate (A), it is necessary to adjust the atmosphere of water vapor and carbon dioxide in the air inside the chamber at predetermined intervals. However, in this sealed measurement method involving the adjustment of the atmosphere, it is difficult to easily and continuously measure the biological information of the plant body.

In view of this situation, a measurement method using a ventilation chamber has also been proposed. In this ventilation method, outside air is introduced into a ventilation chamber that covers all or part of the plant body, and the water vapor and carbon dioxide concentrations at the inlet and outlet of the chamber are obtained by a sensor, and then the transpiration rate (E) and the carbon dioxide assimilation rate (A) of the plant body is measured from the difference in the water vapor or carbon dioxide concentrations between the inlet and outlet. Therefore, in the ventilation measurement, it is possible to measure biological information from the plant body without adjusting the atmosphere inside the chamber at predetermined intervals. However, in order to measure biological information of a plant using the ventilation method, the flow rate of air supplied to the chamber must be obtained as shown in the above formulas (3) and (4), and a gas flow control device or the like must be used, so that it is difficult to actually measure biological information of the plant body easily and continuously.

The present invention has been made in consideration of the above problems, and its object is to provide a biological information measuring device and a method for measuring biological information that can measure biological information of a plant body easily and continuously.

SOLUTION TO PROBLEM

In order to achieve the above-mentioned object, the inventors conducted intensive research and discovered that by using a measuring device having a permeable membrane in one part that has permeation resistance to water vapor or carbon dioxide, the biological information of a plant body can be measured easily and continuously, and thus completed the present invention.

Specifically, the biological information measuring device disclosed herein comprises a container for covering at least a part of a leaf of a plant, and a sensor for measuring the concentration of at least one of water vapor and carbon dioxide inside and outside the container, and is characterized in that at least a part of the wall constituting the container is constituted by a permeable membrane having permeation resistance to water vapor or carbon dioxide.

According to the biological information measuring device of the present disclosure, by covering a part of a leaf of a plant with a container, a space is formed between the container and the leaf, and the concentration of at least one of water vapor and carbon dioxide in the air inside and outside the space can be measured by a sensor. The concentration of at least one of water vapor and carbon dioxide measured in this way can be used to calculate the biological information of the plant body from the permeation resistance of the permeable membrane. Therefore, in the biological information measuring device of the present invention, it is not necessary to obtain the flow rate of air in the container or to use a gas flow control device to measure the biological information. In addition, since a part of the wall constituting the container is a permeable membrane and the air inside the space is open to the outside air through the permeable membrane, there is no risk of the water vapor in the air in the space being saturated or the carbon dioxide being depleted. Therefore, the biological information measuring device of the present invention can easily and continuously measure the biological information of the plant body. The same is true in the case where the leaf or the entire plant is covered with a container, and the concentration of at least one of water vapor and carbon dioxide in the air inside and outside the space of the container can be measured by a sensor. The concentration of at least one of water vapor and carbon dioxide measured in this way can be used to calculate the biological information of the plant body from the permeation resistance of the permeable membrane. Therefore, the biological information measuring device according to the present invention makes it possible to measure biological information of a plant body easily and continuously.

The biological information measuring device according to the present disclosure may further include a calculator for calculating at least one of the transpiration rate and the carbon dioxide assimilation rate in a leaf of a plant based on the difference in concentration of at least one of water vapor and carbon dioxide between inside and outside the container and the permeation resistance.

In this way, at least one of the plant's biological information, i.e., the transpiration rate and the carbon dioxide assimilation rate, can be calculated.

The biological information measuring device according to the present disclosure is characterized in that it comprises a permeable membrane having permeation resistance to water vapor or carbon dioxide, a sealing layer provided on the surface of the permeable membrane so as to surround a predetermined region of the surface and adhering a leaf of a plant to the permeable membrane, and a sensor provided inside and outside the predetermined region of the surface of the permeable membrane for measuring the concentration of at least one of water vapor and carbon dioxide.

According to the biological information measuring device of the present disclosure, a sealing layer provided on the surface of a permeable membrane so as to surround a predetermined region of the surface is adhered to a leaf of a plant, to form a space between the permeable membrane, the sealing layer and the leaf, and the concentration of at least one of water vapor and carbon dioxide in the air inside and outside the space can be measured by sensors provided inside and outside the predetermined region of the surface of the permeable membrane. The concentration of at least one of water vapor and carbon dioxide measured in this manner can be used to calculate the biological information of the plant body from the permeation resistance of the permeable membrane. Therefore, in the biological information measuring device of the present invention, it is not necessary to obtain the flow rate of air in the space or to use a gas flow control device to measure the biological information. In addition, since the air inside the space is open to the outside air through the permeable membrane, there is no risk of the water vapor in the air in the space becoming saturated or the carbon dioxide becoming depleted. Therefore, the biological information measuring device of the present invention can measure the biological information of the plant body easily and continuously.

The biological information measuring device according to the present disclosure may further include a calculator for calculating at least one of the transpiration rate and the carbon dioxide assimilation rate in the leaf of the plant based on the concentration difference of at least one of water vapor and carbon dioxide between inside and outside a space formed by the permeable membrane, the sealing layer and the leaf of the plant in the predetermined region of the permeable membrane, and the permeation resistance.

A biological information measuring device including the above-described calculator can calculate at least one of the biological information of a plant, that is, the transpiration rate and the carbon dioxide assimilation rate.

In the biological information measuring device according to the present disclosure, the permeable membrane may have a plurality of pores.

In this way, water vapor or carbon dioxide can pass through while maintaining a certain degree of permeation resistance, and the air inside the space can be opened to the outside air. In addition, since the difference in concentration of water vapor or carbon dioxide between the air inside the space and the outside air, which is generated by the biological activities of the plant such as transpiration and photosynthesis, can be maintained, this difference in concentration can be used to measure biological information of the plant.

The method for measuring biological information disclosed herein is characterized by comprising a step of covering at least a part of a leaf of a plant with a container at least a part of which is constituted by a permeable membrane having permeation resistance to water vapor or carbon dioxide, to form a space between the container and the leaf, or storing a leaf of a plant in the space of the container, a step of measuring the concentration of at least one of water vapor and carbon dioxide inside and outside the space, and a step of calculating at least one of the transpiration rate and the carbon dioxide assimilation rate in a leaf of a plant based on the concentration difference measured between the inside and outside of the space of at least one of water vapor and carbon dioxide and the permeability constitution.

According to the method for measuring biological information of the present disclosure, by covering a part of the leaf of a plant with a container, a space is formed between the container and the leaf, and the concentration of at least one of water vapor and carbon dioxide in the air inside and outside the space can be measured. The concentration of at least one of water vapor and carbon dioxide measured in this way can be used to calculate the biological information of the plant body from the permeation resistance of the permeable membrane to water vapor or carbon dioxide. Therefore, in the method for measuring biological information of the present invention, it is not necessary to obtain the flow rate of air in the container or to use a gas flow control device for measuring the biological information. In addition, since a part of the wall constituting the container is a permeable membrane and the air inside the space is open to the outside air through the permeable membrane, there is no risk of the water vapor in the air in the space being saturated or the carbon dioxide being depleted. Therefore, the method for measuring biological information of the present invention can easily and continuously measure the biological information of the plant body. The same is true in the case where the leaf or the entire plant are covered with a container, and the concentration of at least one of water vapor and carbon dioxide in the air inside and outside the space of the container can be measured. The concentration of at least one of water vapor and carbon dioxide measured in this way can be used to calculate the biological information of the plant body from the permeation resistance of the permeable membrane. Therefore, the biological information measuring device according to the present invention makes it possible to measure biological information of a plant body easily and continuously.

The method for measuring biological information disclosed herein may include a step of further covering the back side of a leaf of a plant covered by the container with a gas barrier membrane, or a step of covering the back side of a leaf of a plant with another container, at least a part of which is constituted by a permeable membrane that has permeation resistance to water vapor or carbon dioxide, to further form a space between the other container and the back side of the leaf.

According to the method for measuring biological information including the above steps, it is possible to measure biological information of plants even if the plants have two-sided stomatal leaves.

The method for measuring biological information according to the present disclosure is characterized by comprising a step of using a film constituted by a permeable membrane having permeation resistance to water vapor or carbon dioxide and the sealing layer provided on the surface of the permeable membrane so as to surround a predetermined region of the surface, to adhere at least a part of a leaf of a plant to the sealing layer of the film, thereby forming a space between the film and the leaf in the predetermined region of the film, a step of measuring the concentration of at least one of water vapor and carbon dioxide inside and outside the space, and a step of calculating at least one of the transpiration rate and the carbon dioxide assimilation rate in the leaf of the plant based on the concentration difference measured of at least one of water vapor and carbon dioxide between the inside and outside of the space and the permeation resistance.

According to the method for measuring biological information of the present disclosure, a space is formed by the film and the leaf by adhering the sealing layer provided so as to surround a predetermined region of the surface of the permeable membrane in the film and the leaf, and the concentration of at least one of water vapor and carbon dioxide inside and outside the space can be measured. The concentration of at least one of water vapor and carbon dioxide measured in this way can be used to calculate the biological information of the plant body from the permeation resistance of the permeable membrane to water vapor or carbon dioxide. Therefore, in the method for measuring biological information of the present invention, it is not necessary to obtain the air flow rate of the space or to use a gas flow control device to measure the biological information. In addition, since the air inside the space is open to the outside air through the permeable membrane, there is no risk of the water vapor in the air in the space becoming saturated or the carbon dioxide becoming depleted. Therefore, the method for measuring biological information of the present invention can measure the biological information of the plant body easily and continuously.

The method for measuring biological information disclosed herein may include a step of further covering the back side of a leaf of a plant covered by the film with a gas barrier membrane, or a step of using another film constituted by a permeable membrane having permeation resistance to water vapor or carbon dioxide and a sealing layer provided on the surface of the permeable membrane so as to surround a predetermined region of the surface, to adhere the back side of the leaf of the plant to the sealing layer of the other film, thereby further forming a space between the other film and the back side of the leaf in the predetermined region of the other film.

According to the method for measuring biological information including the above steps, it is possible to measure biological information of plants even if the plants have two-sided stomatal leaves.

In the biological information measuring method according to the present disclosure, the permeable membrane may have a plurality of pores.

In this way, water vapor or carbon dioxide can pass through while maintaining a certain degree of permeation resistance, so that the air inside the space can be opened to the outside air. In addition, the permeable membrane having a plurality of pores can maintain the difference in concentration of water vapor or carbon dioxide between the air inside the space and the outside air that occurs due to the biological activities of the plant, such as transpiration and photosynthesis, so that this concentration difference can be used to measure biological information of the plant.

ADVANTAGEOUS EFFECTS OF INVENTION

According to the biological information measuring device and the biological information measuring method of the present invention, biological information of a plant body can be measured easily and continuously.

BRIEF DESCRIPTION OF DRAWINGS

[FIG. 1]FIG. 1 is a side view showing a biological information measuring device according to an embodiment of the present invention.

[FIG. 2]FIG. 2 is a side view showing a biological information measuring device according to one embodiment of the present invention.

[FIG. 3]FIG. 3 is a side view showing an example of use of the biological information measuring device according to one embodiment of the present invention.

[FIG. 4]FIG. 4 is a side view showing an example of use of the biological information measuring device according to one embodiment of the present invention.

[FIG. 5]FIG. 5 is a schematic view showing a model of an example of calculating biological information according to an embodiment of the present invention.

[FIG. 6]FIG. 6 is a side view showing a biological information measuring device according to another embodiment of the present invention.

[FIG. 7]FIG. 7 is a side view showing an example of use of a biological information measuring device according to another embodiment of the present invention.

[FIG. 8]FIG. 8 shows an example of measurement of biological information according to one embodiment of the present invention, in which FIG. 8(a) is a schematic view showing an example of measurement when the leaf of a plant is one-sided stomatal and FIG. 8(b) is a longitudinal cross-sectional view of the container and the leaf during measurement.

[FIG. 9]FIG. 9 is a view showing an example of measurement of biological information according to one embodiment of the present invention, in which FIG. 9(a) is a schematic view showing an example of measurement when the leaf of a plant is two-sided stomatal, and FIG. 9(b) is a longitudinal cross-sectional view of a gas barrier membrane, a leaf, and a container during measurement.

[FIG. 10]FIG. 10 is a view showing an example of measurement of biological information according to one embodiment of the present invention, in which FIG. 10(a) is a schematic view showing another measurement example in the case where the leaf of a plant is two-sided stomatal, and FIG. 10(b) is a longitudinal cross-sectional view of two containers and the leaf during measurement.

[FIG. 11]FIG. 11 is a view showing an example of measurement of biological information according to one embodiment of the present invention, in which FIG. 11(a) is a schematic view showing an example of measurement applicable to both one-sided and two-sided stomatal leaf of a plant, and FIG. 11(b) is a longitudinal cross-sectional view of a container and a leaf during measurement.

[FIG. 12]FIG. 12 is a schematic view showing a biological information measuring device according to an embodiment of the present invention.

[FIG. 13]FIG. 13 is a graph showing the measurement results of biological information in Example 1-1, in which FIG. 13(a) shows the light responsiveness of the transpiration rate (E) and carbon dioxide assimilation rate (A) of grape leaf, lemon leaf, and cherry leaf, and FIG. 13(b) shows the carbon dioxide responsiveness of the transpiration rate (E) and carbon dioxide assimilation rate (A) of grape leaf, lemon leaf, and cherry leaf.

[FIG. 14]FIG. 14 is a graph showing the measurement results of biological information in Example 1-2, and shows the light responsiveness of the transpiration rate (E) and the carbon dioxide assimilation rate (A) of grape leaf.

[FIG. 15]FIG. 15 is a graph showing the measurement results of biological information in Examples 1-3, in which FIG. 15(a) shows the light responsiveness of the transpiration rate (E) and carbon dioxide assimilation rate (A) of sunflower leaf, and FIG. 15(b) shows the carbon dioxide responsiveness of the transpiration rate (E) and carbon dioxide assimilation rate (A) of sunflower leaf.

[FIG. 16]FIG. 16 is a graph showing the measurement results of biological information in Example 1-4, showing the light responsiveness of the transpiration rate (E) and the carbon dioxide assimilation rate (A) of strawberry leaf and sunflower leaf.

[FIG. 17]FIG. 17 is a graph showing the measurement results of biological information in Example 2, where FIG. 17(a) shows the light responsiveness of the transpiration rate (E) and carbon dioxide assimilation rate (A) of sunflower leaf when permeable membranes with different mesh numbers are used, and FIG. 17(b) shows the carbon dioxide responsiveness of the transpiration rate (E) and carbon dioxide assimilation rate (A) of sunflower leaf when permeable membranes with different mesh numbers are used.

[FIG. 18]FIG. 18 is a graph showing the measurement results of biological information in Example 3, in which FIG. 18(a) shows the diffusion conductance ($g_{fw}$) of the permeable membrane to water vapor and the diffusion conductance ($g_{fc}$) to carbon dioxide in $T_{air}$, and FIG. 18(b) shows the carbon dioxide assimilation rate (A) in $T_{leaf}$.

[FIG. 19]FIG. 19 is a graph showing the measurement results of biological information in Example 4. The left side shows the SPAD value measured for each leaf position using a chlorophyll meter SPAD-502Plus (manufactured by Konica Minolta), and the right side shows the carbon dioxide assimilation rate (A) measured for each leaf position using a biological information measuring device using a permeable membrane and an LI-6800.

[FIG. 20]FIG. 20 is a graph showing the measurement results of biological information relating to Example 5-1, and shows the light responsiveness of the transpiration rate (E) and the carbon dioxide assimilation rate (A) before irrigation was stopped (solid line), 3 days after irrigation was stopped (dotted line), and 4 days after irrigation was stopped (dashed line).

[FIG. 21]FIG. 21 is a graph showing the measurement results of biological information for Example 5-2, where FIG. 21(a) shows the change in sunflower pot weight (g) over time and the change in evapotranspiration (g) of sunflower and pot soil over time, and FIG. 21(b) shows the change in sunflower leaf transpiration rate ($E_{NEW}$) and carbon dioxide assimilation rate ($A_{NEW}$) over time measured by a biological information measuring device using a permeable membrane.

[FIG. 22]FIG. 22 is a schematic view showing a sealed chamber equipped with a sensor, which is an example of a conventional biological information measuring device.

[FIG. 23]FIG. 23 is a schematic view showing a ventilated chamber equipped with a sensor, which is an example of a conventional biological information measuring device.

[FIG. 24]FIG. 24 is a schematic view showing a model of an example of calculation of the transpiration rate ($E_{STD}$) and the carbon dioxide assimilation rate ($A_{STD}$) based on ventilation-type measurements.

DESCRIPTION OF EMBODIMENTS

Hereinafter, embodiments of the present invention will be described with reference to the drawings. The following preferred embodiments are merely exemplary in nature and are not intended to limit the present invention, its application method, or its uses.

< Biological Information Measuring Device>

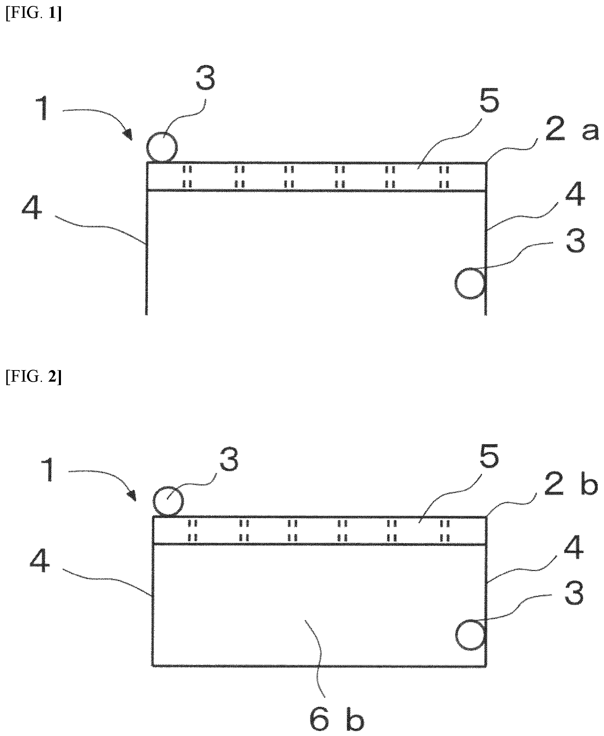

A biological information measuring device (1) according to one embodiment of the present invention will be described below with reference to FIGS. 1 to 5.

As shown in FIGS. 1 and 2, a biological information measuring device (1) according to one embodiment of the present invention comprises containers (2a, 2b) for covering at least a part of a leaf (8) of a plant, and sensors (3) provided on the inside and outside of the containers (2a, 2b) for measuring the concentration of at least one of water vapor and carbon dioxide, and at least a part of a wall (4) constituting the containers (2a, 2b) is constituted a permeable membrane (5) having permeation resistance to water vapor or carbon dioxide.

For example, the biological information measuring device (1) may be one in which a container (2a) has an opening as shown in FIG. 1, or one in which a container (2b) has no opening (but may have a small pore that allows a part of the plant to pass through) and has a closed space (6b) inside the container (2b) as shown in FIG. 2. Such a biological information measuring device (1) can be used according to the size and shape of the plant, and can measure biological information without damaging the plant.

As shown in FIGS. 1 and 2, the containers (2a, 2b) include at least a wall (4) that is impermeable to gases such as water vapor or carbon dioxide, and a permeable membrane (5) that has permeation resistance to water vapor or carbon dioxide, and at least a part of the wall (4) is constituted by the permeable membrane (5). For example, the containers (2a, 2b) may be glass containers or plastic containers in which a part of the wall (4) of the container is constituted by the permeable membrane (5). The number of permeable membranes (5) is not particularly limited, and may be two or more.

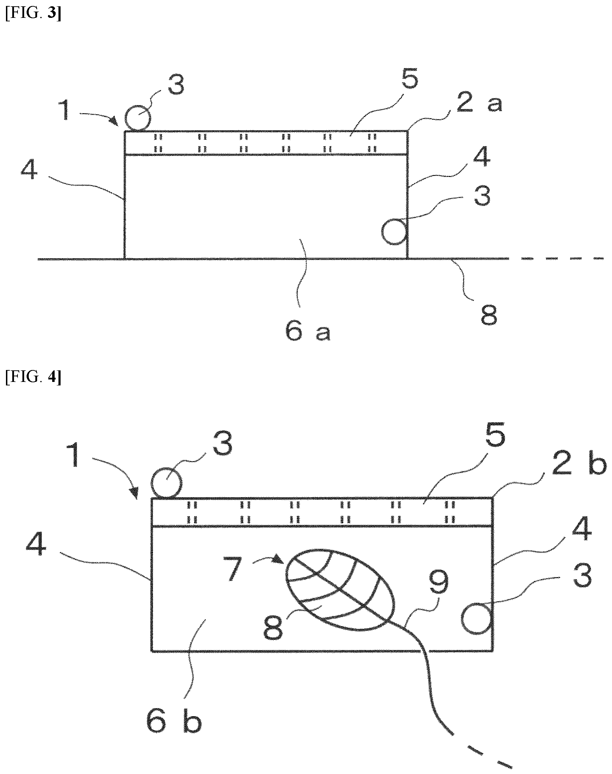

As shown in FIGS. 1 and 2, the sensor (3) is not particularly limited as long as it measures the concentration of either water vapor or carbon dioxide, and a commercially available product can be used. For example, the sensor (3) is provided on the inside and outside of the containers (2a, 2b), and is not particularly limited, but may be, for example, a water vapor sensor on the inside and outside of the containers (2a, 2b), a carbon dioxide sensor on the inside and outside of the containers (2a, 2b), or both a water vapor sensor and a carbon dioxide sensor on the inside of the containers (2a, 2b) and both a water vapor sensor and a carbon dioxide sensor on the outside. By providing the sensor (3) in such a position on the containers (2a, 2b), it is possible to measure the concentration difference of at least one of water vapor and carbon dioxide between the space (6a) formed by the container (2a) and the leaf (8) and the outside air (see FIG. 3), or between the space (6b) of the container (2b) and the outside air. In addition, the outer sensor (3) may be located at a position away from the containers (2a, 2b). Furthermore, when measuring biological information, one external sensor (3) may be used for multiple containers (2a, 2b), or one external sensor (3) may be used for all containers (2a, 2b). In particular, when measuring biological information for multiple plant bodies, it is preferable that the containers (2a, 2b) are provided with a representative external sensor (3). As described above, the sensor (3) may be any sensor capable of measuring the concentration of either water vapor or carbon dioxide, and for example, a commercially available gas analyzer may be used. Examples of such gas analyzers include LI-7000 (manufactured by LI-COR). In addition, a leaf temperature sensor that measures the surface temperature of a leaf (8) of a plant may also be used as the sensor (3). In this way, the environmental conditions can be measured by the measured leaf temperature, and biological information such as the leaf gas diffusion conductance, which is a determining factor of the transpiration rate or the carbon dioxide assimilation rate, can be obtained. The sensor (3) does not need to be provided as a whole sensor device on the inside and outside of the containers (2a, 2b), but it is sufficient that a measurement sensitive part for detecting at least one of the concentrations of water vapor and carbon dioxide is provided on the inside and outside of the containers (2a, 2b), respectively. In other words, the sensor (3) is required to be disposed in a form capable of measuring the concentration of either water vapor or carbon dioxide on the inside and outside of the containers (2a, 2b).

As shown in FIGS. 1 and 2, the wall (4) of the container is not particularly limited as long as it is impermeable to gases such as water vapor or carbon dioxide, and examples of the material include glass and plastic.

As shown in FIG. 1 and FIG. 2, the permeable membrane (5) can be appropriately selected from membranes having permeation resistance to water vapor or carbon dioxide. In addition, the permeable membrane (5) preferably allows moderate permeation of gases such as water vapor or carbon dioxide, and preferably has a plurality of pores. For example, a nylon mesh can be used as the permeable membrane (5). In this way, for example, the concentration difference of water vapor or carbon dioxide generated by the transpiration or photosynthesis of the plant between the inside and outside of the space (6a) formed by the container (2a) and the leaf (8) or between the inside and outside of the space (6b) of the container (2b) can be maintained, which is suitable for measuring the biological information of a leaf (8) of a plant and allows a leaf (8) of a plant to be exposed to the outside air. In particular, since the permeable membrane (5) needs to open the inside and outside of the containers (2a, 2b) while having a predetermined permeation resistance, the opening ratio of the permeable membrane (5) having a plurality of pores is preferably 1% to 50%, more preferably 5% to 50%, and even more preferably 10% to 50%. Furthermore, the diffusion conductance of the permeable membrane (5) for carbon dioxide is preferably 1200 mmolm$^{-2}$s$^{-1}$ or less, and the diffusion conductance of the permeable membrane (5) for water vapor is preferably 1100 mmolm$^{-2}$s$^{-1}$ or less. In these cases, the concentration difference of carbon dioxide or water vapor through the permeable membrane (5) can be maintained large, and the measurement accuracy can be improved. Note that these diffusion conductances cannot be measured when they are 0, so the lower limit of the diffusion conductance is a value more than 0.

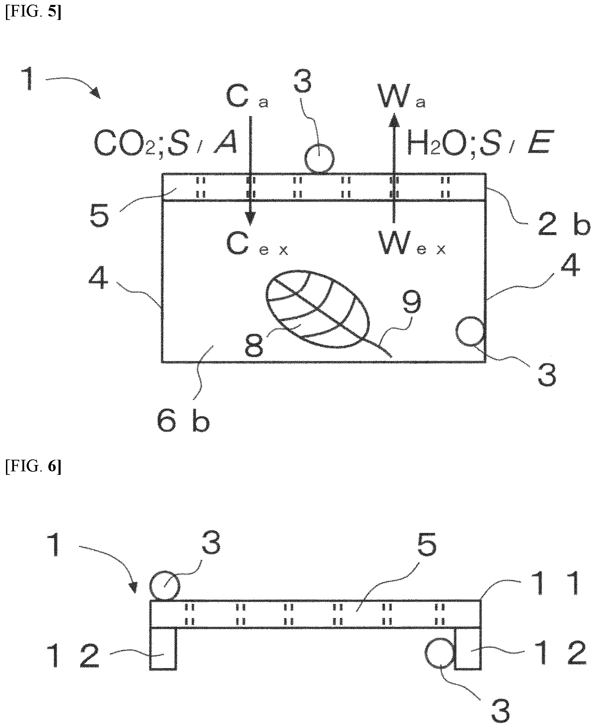

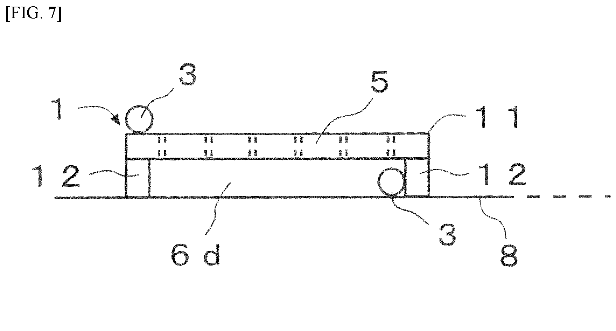

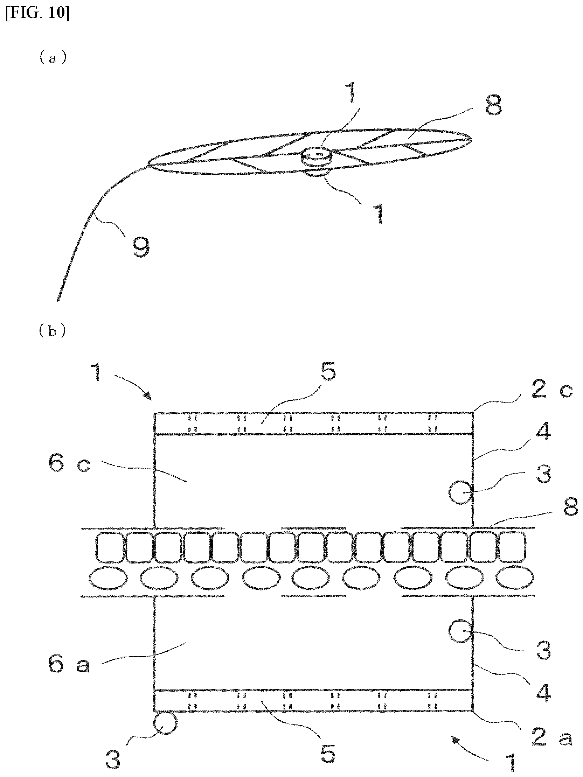

In this specification, the meaning of "spaces (6a, 6b, 6c, 6d)" is used according to the type of container (2a, 2b, 2c) or film (11) used. Specifically, in the case where the container (2a) shown in FIG. 1 has an opening, the space (6a) refers to the space formed by the container (2a) and a leaf (8) of a plant as shown in FIG. 3. In addition, as will be described later, in the case where the container (2c) having an opening is used, the space (6c) refers to the space formed by the container (2c) and a leaf (8) of a plant as shown in FIG. 10(b). On the other hand, in the case where the container (2b) shown in FIG. 2 does not have an opening, the space (6b) refers to the space inside the container (2b) as shown in FIG. 4. In addition, as shown in FIG. 6, in the case of the film (11) described later, the space (6d) refers to the space formed by the permeable membrane (5), the sealing layer (12), and a leaf (8) of a plant as shown in FIG. 7. In such spaces (6a, 6b, 6c, 6d), a difference in concentration of water vapor or carbon dioxide is maintained between the inside and outside of the spaces due to transpiration, photosynthesis, etc. by plants. Therefore, it becomes possible to calculate the transpiration rate or the carbon dioxide assimilation rate, etc., based on the difference in concentration of at least one of water vapor and carbon dioxide between the inside and outside of the spaces (6a, 6b, 6c, 6d), and it becomes possible to measure biological information of the plants.

The type and size of the plant (7) to which the biological information measuring device (1) is applied are not particularly limited. The leaf (8) of the plant may be either one-sided or two-sided stomatal. For example, examples of the leaf (8) of the plant that is one-sided stomatal include grape leaf, lemon leaf, and cherry leaf, and examples of the leaf (8) of the plant that is two-sided stomatal include sunflower leaf and strawberry leaf.

The biological information of the plant measured by the biological information measuring device (1) is not particularly limited, and examples thereof include the transpiration rate and the carbon dioxide assimilation rate. The transpiration rate and the carbon dioxide assimilation rate may be expressed for the whole plant or for a leaf of a plant. The biological information of the plant may be biological information calculated based on the above-mentioned transpiration rate and the carbon dioxide assimilation rate, and examples thereof include the irrigation status, fertilization status (nutritional state), aging status, and environmental stress status of the plant. In addition, the fertilization status of the plant has been conventionally estimated by measuring the chlorophyll content of a leaf of a plant using a chlorophyll meter, for example, and using the chlorophyll content as an index. On the other hand, the biological information measuring device (1) according to the present embodiment measures the carbon dioxide assimilation rate (A), and can estimate the fertilization status of the plant using this as an index, and can evaluate the biological information of the plant, including the aging status of the plant.

Hereinafter, the calculation of the plant's biological information using the biological information measuring device (1) of the present embodiment will be specifically described. The biological information of the plant here is the transpiration rate and the carbon dioxide assimilation rate in leaves, and as shown in FIG. 5, a model in which the leaf (8) is stored in the container (2b) of the biological information measuring device (1) of the present embodiment will be described. First, the water vapor concentration ($W_{ex}$) and the carbon dioxide concentration ($C_{ex}$) in the space (6b) are measured by the sensor (3) inside the container (2b), and the water vapor concentration ($W_a$) and the carbon dioxide concentration ($C_a$) outside the space (6b) are measured by the sensor (3) outside the container (2b). Here, although not shown, in the space (6b), water vapor is released from the leaf (8) into the space (6b) by transpiration, and carbon dioxide is taken up from the space (6b) into the leaf (8) by photosynthesis. Therefore, according to Fick's first law, the relationship between the transpiration rate (E) and the water vapor concentration difference ($W_{ex}$ - $W_a$) between the inside and outside of the space (6b) is expressed by the following formula (5).

[Mathematical Formula 3]

$$S_l E = S_f \left[ g_{fw}(W_{ex} - W_a) + X_{fw} \right] \qquad (5)$$

In formula (5), $S_l$ represents the area of the measurement leaf, $S_f$ represents the area of the permeable membrane, $g_{fw}$ represents the diffusion conductance of the permeable membrane to water vapor, and $X_{fw}$ represents the interaction between water molecules and carbon dioxide molecules in the permeable membrane.

Next, when the above formula (5) is rearranged to reflect the transpiration rate (E), the following formula (6) is obtained.

[Mathematical Formula 4]

$$E = \frac{S_f}{S_l} \left[ g_{fw}(W_{ex} - W_a) + X_{fw} \right] \qquad (6)$$

Furthermore, if the carbon dioxide assimilation rate (A) is expressed in the same manner as the transpiration rate (E), the following formula (7) is obtained.

[Mathematical Formula 5]

$$A = \frac{S_f}{S_l} \left[ g_{fc}(C_a - C_{ex}) - X_{fc} \right] \qquad (7)$$

In formula (7), $S_f$ represents the area of the permeable membrane, $S_l$ represents the area of the measurement leaf, $g_{fc}$ represents the diffusion conductance of the permeable membrane to carbon dioxide, and $X_{fc}$ represents the interaction between water molecules and carbon dioxide molecules in the permeable membrane.

On the other hand, $X_{fw}$ and $X_{fc}$ can be estimated by the following formulas (8) and (9), respectively.

[Mathematical Formula 6]

$$X_{fw} = 0.5E(W_{ex} + W_a) \qquad (8)$$

$$X_{fc} = 0.5E(C_a + C_{ex}) \qquad (9)$$

Furthermore, when the influence of $X_{fw}$ and $X_{fc}$ is small enough to be ignored, the above formulas (6) and (7) are simplified to the following formulas (10) and (11).

[Mathematical Formula 7]

$$E = \frac{S_f}{S_l} g_{fw}(W_{ex} - W_a) \qquad (10)$$

$$A = \frac{S_f}{S_l} g_{fc}(C_a - C_{ex}) \qquad (11)$$

Furthermore, when the area of the permeable membrane ($S_f$) and the area of the measurement leaf ($S_l$) are the same, the above formulas (10) and (11) can be simplified to the following formulas (12) and (13).

[Mathematical Formula 8]

$$E = g_{fw}(W_{ex} - W_a) \qquad (12)$$

$$A = g_{fc}(C_a - C_{ex}) \qquad (13)$$

Therefore, the transpiration rate (E) of a leaf (8) of a plant can be calculated, for example, from the above formula (12). More specifically, the transpiration rate (E) can be calculated by multiplying the diffusion conductance ($g_{fw}$), which is the reciprocal of the diffusion resistance of the permeable membrane (5) to water vapor, by the difference in water vapor concentration ($W_{ex}$ - $W_a$) between the inside and outside of the space (6b) of the container (2b). In this specification, the "diffusion conductance (g)" refers to the ease of diffusion of molecules in a system and is expressed by the reciprocal of the diffusion resistance. The transpiration rate (E) of a leaf (8) of a plant may be calculated from the above formula (6) or formula (10). The diffusion conductance ($g_{fw}$), which represents the ease of diffusion of water vapor through the permeable membrane (5), can be a value calibrated by an existing measurement method.

The carbon dioxide assimilation rate (A) of a leaf (8) of a plant can be calculated, for example, from the above formula (13). More specifically, the carbon dioxide assimilation rate (A) can be calculated by multiplying the diffusion conductance ($g_{fc}$), which is the reciprocal of the diffusion resistance of the permeable membrane (5) to carbon dioxide, by the difference in carbon dioxide concentration ($C_a$ - $C_{ex}$) between the inside and outside of the space (6b) of the container (2b). The carbon dioxide assimilation rate (A) of a leaf (8) of a plant may be calculated from the above formula (7) or formula (11). The diffusion conductance ($g_{fc}$), which indicates the ease of diffusion of carbon dioxide in the permeable membrane (5), can be a value calibrated by an existing measurement method.

In this specification, the calculation of the transpiration rate (E) and the carbon dioxide assimilation rate (A) of a leaf (8) of a plant when a part of the leaf is covered with the container (2a) of the biological information measuring device (1) has an opening is not described in detail, but it is possible to calculate them using the same formulas as the above-mentioned formulas (5) to (13). In this case, the space (6a) is treated in accordance with the above-mentioned definition.

In the present embodiment, the biological information measuring device (1) may further include a calculator (not shown). Such a calculator is preferably capable of calculating at least one of the transpiration rate and the carbon dioxide assimilation rate in the leaf (8) of the plant based on the difference in concentration of at least one of water vapor and carbon dioxide between inside and outside the containers (2a, 2b, 2c) and the permeation resistance of the permeable membrane (5).

In the present embodiment, the biological information measuring device (1) may further include a wireless communication device (not shown). For example, when the biological information measuring device (1) includes a wireless communication device, it becomes possible to transmit and receive measured data, and it becomes possible to collectively manage biological information of plants at a cultivation site using a management terminal or the like.

In the present embodiment, the biological information measuring device (1) may further include a display unit (not shown). For example, when the biological information measuring device (1) includes a display unit, it is possible to display the measurement results of the biological information of the plant.

In the present embodiment, the biological information measuring device (1) may be provided with any constitution that is effective for measuring the biological information of a plant. Such a constitution may be appropriately used as long as it is commonly used by those skilled in the art, and may include, for example, a fan that stirs the air inside the spaces (6a, 6b).

According to the biological information measuring device (1) as shown in FIG. 1, a part of the leaf (8) of a plant is covered with a container (2a) to form a space (6a) between the container (2a) and the leaf (8), and the concentration of at least one of water vapor and carbon dioxide in the air inside and outside the space (6a) can be measured by sensors (3) provided inside and outside the container (2a) (see FIG. 3). The concentration of either water vapor or carbon dioxide measured in this manner can be used to calculate the biological information of the plant body from the permeation resistance of the permeable membrane (5). Therefore, in the biological information measuring device (1) according to the present embodiment, it is not necessary to obtain the flow rate of the air in the container (2a) or to use a gas flow control device to measure the biological information. In addition, a part of the wall (4) constituting the container (2a) is a permeable membrane (5), and the air inside the space (6a) is open to the outside air through the permeable membrane (5), so there is no risk of the water vapor in the air in the space (6a) becoming saturated or the carbon dioxide becoming depleted. Therefore, the biological information measuring device (1) according to the present invention makes it possible to measure biological information of a plant body easily and continuously.

In addition, with the biological information measuring device (1) as shown in FIG. 2, the leaf (8) or the entire plant (not shown) are covered with a container (2b), and the concentration of at least one of water vapor and carbon dioxide in the space (6b) of the container (2b) and in the air outside the space can be measured by sensors (3) provided inside and outside the container (2b) (see FIG. 4). The concentration of at least one of water vapor and carbon dioxide thus measured can be used to calculate the biological information of the plant body from the permeation resistance of the permeable membrane (5). Therefore, the biological information measuring device (1) according to the present embodiment can measure the biological information of the plant body easily and continuously.

The method for producing the biological information measuring device (1) according to the present embodiment is not particularly limited, but for example, the biological information measuring device (1) can be manufactured by preparing containers (2a, 2b), replacing a part of the wall (4) of the containers (2a, 2b) with a permeable membrane (5), and attaching the sensor (3) to the inside and outside of the containers (2a, 2b). However, the producing method is not limited to this producing example.

A suitable example of use of the biological information measuring device (1) according to the present embodiment will be described in detail in the biological information measuring method described below, but the use is not limited to this example.

A biological information measuring device (1) according to another embodiment of the present invention will be described below with reference to FIGS. 6 and 7.

As shown in FIG. 6, a biological information measuring device (1) according to another embodiment of the present invention may be a film (11) including a permeable membrane (5) having permeation resistance to water vapor or carbon dioxide, a sealing layer (12) provided on the surface of the permeable membrane (5) so as to surround a predetermined region of the surface and for adhering a leaf (8) of a plant and the permeable membrane (5) (see FIG. 7), and a sensor (3) provided inside and outside the predetermined region on the surface of the permeable membrane (5) for measuring the concentration of at least one of water vapor and carbon dioxide. Note that, in FIG. 6, the biological information measuring device (1) is illustrated with a sensor (3) provided on the surface of the sealing layer (12) as a sensor within the predetermined region, and a sensor (3) provided on the surface of the permeable membrane (5) as a sensor outside the predetermined region, but is not limited thereto.

As shown in FIG. 7, the sealing layer (12) is used to adhere the permeable membrane (5) to the surface of a leaf (8) of a plant, for example, and thus a space (6d) can be formed between the permeable membrane (5), the sealing layer (12), and a leaf (8) of a plant. The type of the sealing layer (12) is not particularly limited as long as it fulfills the above-mentioned function. In addition, the sealing layer (12) may further include a protective layer (not shown) for protecting the sealing force of the sealing layer (12) before adhering to a leaf (8) of a plant.

As shown in FIG. 7, the calculation of plant biological information, such as the transpiration rate (E) or the carbon dioxide assimilation rate (A), using the biological information measuring device (1) of the present embodiment can be performed in the same manner as in the case of the container (2a) described above. Therefore, the calculation of biological information will not be described in detail in the present embodiment.

In the present embodiment, the biological information measuring device (1) may further include a calculator (not shown). Such a calculator is preferably capable of calculating at least one of the transpiration rate and the carbon dioxide assimilation rate in a leaf (8) of a plant based on the difference in concentration of at least one of water vapor and carbon dioxide between the inside and outside of the space (6d) formed by the permeable membrane (5), the sealing layer (12), and a leaf (8) of a plant in a predetermined region of the permeable membrane (5) and the permeation resistance of the permeable membrane (5).

According to the biological information measuring device (1) as shown in FIG. 6, the sealing layer (12) provided on the surface of the permeable membrane (5) so as to surround a predetermined region of the surface is adhered to a leaf (8) of a plant to form a space (6d) between the permeable membrane (5), the sealing layer (12), and the leaf (8) (see FIG. 7). The sensor (3) provided inside and outside the predetermined region of the surface of the permeable membrane (5) can measure the concentration of at least one of water vapor and carbon dioxide in the air inside and outside the space (6d).

The concentration of at least one of water vapor and carbon dioxide measured in this manner can be used to calculate the biological information of the plant body from the permeation resistance of the permeable membrane (5). Therefore, in the biological information measuring device (1) according to the present embodiment, it is not necessary to obtain the flow rate of air in the space (6d) or to use a gas flow control device in order to measure the biological information. In addition, since the air inside the space (6d) is open to the outside air through the permeable membrane (5), there is no risk of the water vapor in the air in the space (6d) becoming saturated or the carbon dioxide becoming depleted. Therefore, the biological information measuring device (1) according to the present embodiment can measure biological information of a plant body easily and continuously.

The method of producing the biological information measuring device (1) according to the present embodiment is not particularly limited, but may be, for example, by preparing a permeable membrane (5), attaching an sealing layer (12) to the surface of the permeable membrane (5) so as to surround a predetermined region of the surface, and then adhering the sensor (3) to the inside and outside of the predetermined region. However, the method is not limited to this producing example.

A suitable example of use of the biological information measuring device (1) according to the present embodiment will be described in detail in the biological information measuring method described below, but the use is not limited to this example.

<Method of measuring biological information>

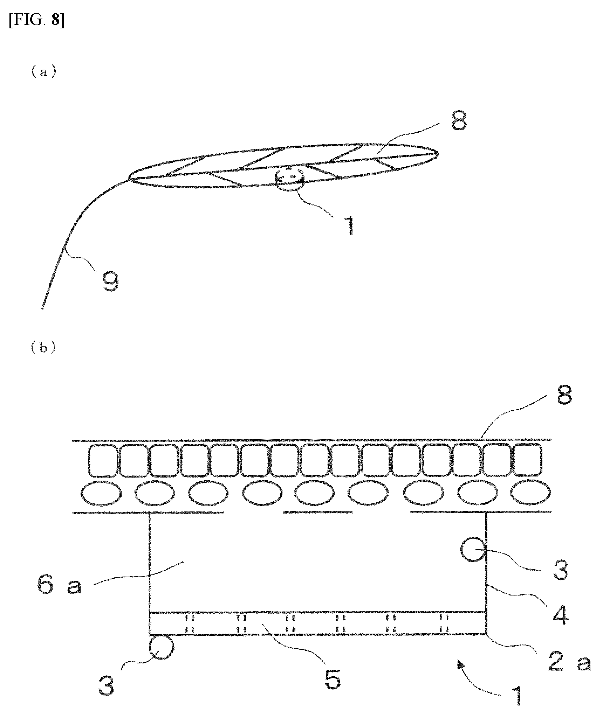

Hereinafter, a method for measuring biological information according to one embodiment of the present invention will be described with reference to FIGS. 8 to 11.

A method for measuring biological information according to one embodiment of the present invention includes the following steps.

A step of covering at least a part of a leaf (8) of a plant with a container (2a, 2b) at least a part of which is constituted by a permeable membrane (5) having permeation resistance to water vapor or carbon dioxide to form a space (6a) between the container (2a) and the leaf (8) or storing a leaf (8) of a plant in the space (6b) of the container (2b); a step of measuring the concentration of at least one of water vapor and carbon dioxide inside and outside the space (6a, 6b); and a step of calculating at least one of the transpiration rate and the carbon dioxide assimilation rate in a leaf (8) of a plant based on the measured concentration difference between the inside and outside of the space (6a, 6b) of at least one of water vapor and carbon dioxide and the permeation resistance of the permeable membrane (5).

In the method for measuring biological information of the present embodiment, the containers (2a, 2b), the sensor (3), the container walls (4), the permeable membrane (5), the space (6a, 6b), the plant (7), and the calculation of the biological information of the plant are as described above and will not be described in detail. In addition, in the method for measuring biological information of the present embodiment, a biological information measuring device (1) including the above-mentioned containers (2a, 2b) can be suitably used.

The method for measuring biological information according to one embodiment of the present invention may further include the following steps.

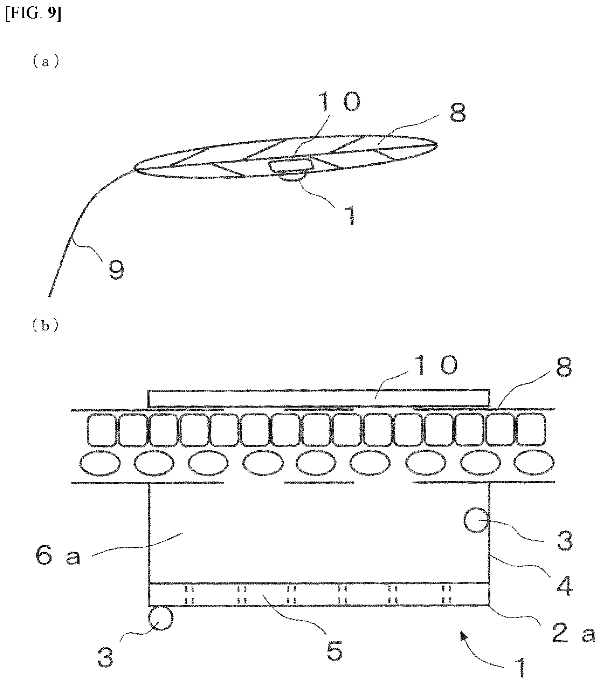

A step of further covering the back side of a leaf (8) of a plant covered by the container (2a) with a gas barrier membrane (10) (see FIG. 9), or a step of covering the back side of a leaf (8) of a plant with another container (2c) at least a part of which is constituted by a permeable membrane (5) having permeation resistance to water vapor or carbon dioxide, thereby further forming a space (6c) between the other container (2c) and the back side of the leaf (8) (see FIG. 10).

As shown in FIGS. 9(a) and (b), the gas barrier membrane (10) may be, for example, a plastic film without pores, such as PROPAFILM (registered trademark).

As shown in FIG. 10(b), the other container (2c) may be, for example, the above-mentioned biological information measuring device (1), or a container equipped with a leaf temperature sensor (not shown) may be used.

If the above-mentioned steps are further included in the present embodiment, the biological information of the plant can be measured even if the plant has a two-sided stomatal leaf (8). Specifically, as shown in FIGS. 9(a) and (b), the stomata on the back side of a leaf (8) of a plant are covered with a gas barrier membrane (10) to prevent the release of water vapor by transpiration or the intake of carbon dioxide by photosynthesis from the stomata on the back side of a leaf (8) of a plant, or as shown in FIGS. 10(a) and (b), the stomata on the back side of a leaf (8) of a plant are covered with a container (2c) at least a part of which is constituted by a permeable film (5), so that the biological information from the stomata on the back side of a leaf (8) of a plant can also be measured.

Hereinafter, a specific example of measuring biological information according to the present embodiment will be described. For example, when a leaf (8) of a plant is one-sided stomatal, the biological information is measured as shown in FIGS. 8(a) and (b). First, a container (2a) is prepared, at least a part of which is constituted by a permeable membrane (5) having permeation resistance to water vapor or carbon dioxide and having an opening, and sensors (3) are attached to the inside and outside of the container (2a). Next, the opening of the prepared container (2a) covers a part of the abaxial surface of a leaf (8) of a plant on which the stomata are present, forming a space (6a) between the container (2a) and the leaf (8). Then, the sensor (3) measures the concentration difference of at least one of water vapor and carbon dioxide inside and outside the space (6a). Finally, at least one of the transpiration rate (E) and the carbon dioxide assimilation rate (A) in a leaf (8) of a plant is calculated based on the measured concentration difference of water vapor or carbon dioxide and the permeation resistance, and the biological information of the plant is measured.

Another example of the measurement of biological information according to the present embodiment will be described in detail. For example, when a leaf (8) of a plant has two-sided stomata, the biological information is measured as shown in FIGS. 9(a) and (b). First, a container (2a) is prepared, at least a part of which is constituted by a permeable membrane (5) having permeation resistance to water vapor or carbon dioxide and having an opening, and sensors (3) are attached to the inside and outside of the container (2a). Next, a part of one side of a leaf (8) of a plant is covered by the opening of the prepared container (2a), forming a space (6a) between the container (2a) and the leaf (8). In addition, a part of the opposite side of a leaf (8) of a plant covered by the container (2a) is further covered by a gas barrier membrane (10). In this way, it is possible to prevent the loss of water vapor or carbon dioxide due to transpiration or photosynthesis from the opposite side of a leaf (8) of a plant. Then, the concentration difference of at least one of water vapor and carbon dioxide inside and outside the space (6a) is measured. Finally, at least one of the transpiration rate (E) and the carbon dioxide assimilation rate (A) in a leaf (8) of a plant is calculated based on the measured water vapor or carbon dioxide concentration difference and permeation resistance, and the plant's biological information is measured.

Also, for example, when a leaf (8) of a plant has two-sided stomata, it is possible to measure biological information as shown in FIGS. 10(a) and (b). First, containers (2a, 2c) are prepared, at least a part of which is constituted by a permeable membrane (5) having permeation resistance to water vapor or carbon dioxide and having openings, and a container (2a) with a sensor (3) attached to the inside and outside, and a container (2c) with a sensor (3) attached to the inside are prepared. Next, the opening of the prepared container (2a) covers a part of one side of a leaf (8) of a plant to form a space (6a) between the container (2a) and the leaf (8). Furthermore, the opening of the prepared container (2c) covers a part of the opposite side of a leaf (8) of a plant covered by the container (2a) to form a space (6c) between the container (2c) and the leaf (8). In this way, it is possible to prevent the loss of water vapor or carbon dioxide due to transpiration or photosynthesis from the opposite side of a leaf (8) of a plant. Then, the concentration difference of at least one of water vapor and carbon dioxide between the inside and outside of one of the spaces, i.e., the space (6a) and the space (6c), is measured. Although not particularly limited, the concentration of at least one of water vapor and carbon dioxide outside the space (6c) can be a value measured by the sensor (3) outside the container (2a). Finally, at least one of the transpiration rate (E) and the carbon dioxide assimilation rate (A) in the leaf (8) of the plant is calculated based on the measured concentration difference of water vapor or carbon dioxide and the permeation resistance, and the biological information of the plant is measured.

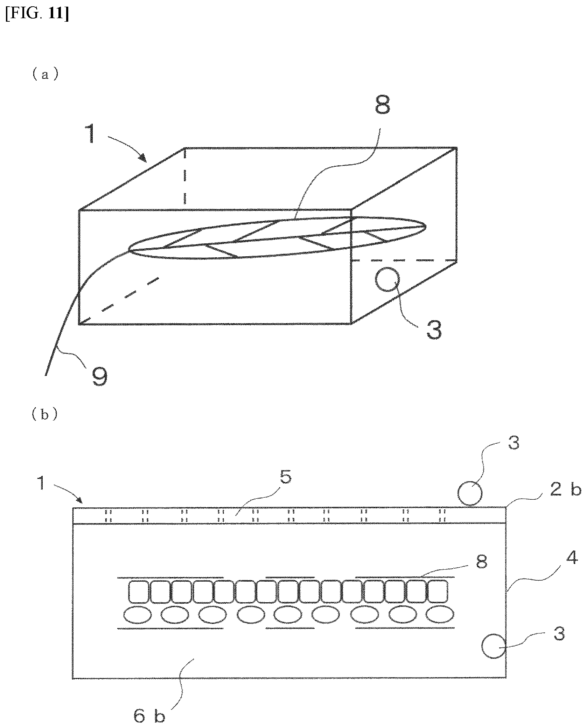

Further, another example of the measurement of biological information according to the present embodiment will be specifically described. In this measurement example, biological information is measured as shown in FIGS. 11(a) and (b), and it can be applied to both the case where a leaf (8) of a plant has one-sided stomata and the case where a leaf (8) of a plant has two-sided stomata. First, a container (2b) is prepared, at least a part of which is constituted by a permeable membrane (5) having permeation resistance to water vapor or carbon dioxide (not shown in FIG. 11(a)) and has no opening, and a sensor (3) is attached to the inside and outside of the container (2b). Next, a leaf (8) of a plant is stored in the space (6b) of the prepared container (2b). At this time, if a part of the wall (4) of the container (2b) is constituted to be openable and closable, it is preferable to easily store a leaf (8) of a plant. In addition, although not shown, it is preferable to use a seal or the like appropriately so that the space (6b) is not directly opened to the outside air by the plant stem (9) during storage. Then, the difference in concentration of at least one of water vapor and carbon dioxide between the inside and outside of the space (6b) of the container (2b) is measured. Finally, at least one of the transpiration rate (E) and the carbon dioxide assimilation rate (A) in the leaf (8) of the plant is calculated based on the measured difference in concentration of water vapor or carbon dioxide and the permeation resistance, thereby measuring biological information of the plant.

The measurement of the concentration of at least one of water vapor and carbon dioxide in the air outside the above-mentioned space (6a, 6b, 6c) is not limited to the method of attaching a sensor (3) to the outside of the container (2a, 2b, 2c) and measuring, but may be performed, for example, by installing an external sensor (3) at a position away from the container (2a, 2b, 2c). In addition, when measuring biological information of multiple plant bodies simultaneously, one external sensor (3) may be used for multiple containers (2a, 2b, 2c), or one external sensor (3) may be used for all containers (2a, 2b, 2c).

That is, according to the method for measuring biological information of the present embodiment, a space (6a) is formed by the container (2a) and the leaf (8) by covering a part of the leaf (8) of the plant with the container (2a), and the concentration of at least one of water vapor and carbon dioxide can be measured inside and outside the space (6a). The concentration of at least one of water vapor and carbon dioxide measured in this way can be used to calculate the biological information of the plant body from the permeation resistance of the permeable membrane (5) to water vapor or carbon dioxide. Therefore, in the method for measuring biological information of the present embodiment, it is not necessary to obtain the flow rate of air in the container (2a) or to use a gas flow control device to measure the biological information. In addition, since a part of the wall (4) constituting the container (2a) is the permeable membrane (5), and the air inside the space (6a) is open to the outside air through the permeable membrane (5), there is no risk of the water vapor in the air in the space (6a) becoming saturated or the carbon dioxide becoming depleted. Therefore, the method for measuring biological information of the embodiment can measure the biological information of the plant body easily and continuously.

Furthermore, according to the method for measuring biological information of the present embodiment, the same applies to the case where the leaf (8) or the entire plant (not shown) are covered with the container (2b), and it is possible to measure the concentration of at least one of water vapor and carbon dioxide inside and outside the space (6b) of the container (2b). The concentration of water vapor or carbon dioxide measured in this manner makes it possible to calculate the biological information of the plant body from the permeation resistance of the permeable membrane (5). Therefore, the method for measuring biological information of the present embodiment makes it possible to easily and continuously measure the biological information of the plant body.

A biological information measuring method according to another embodiment of the present invention will be described below.

Although not shown, a method for measuring biological information according to another embodiment of the present invention may include the following steps.

A step of using a film (11) constituted by a permeable membrane (5) having permeation resistance to water vapor or carbon dioxide and an sealing layer (12) provided on the surface of the permeable membrane (5) so as to surround a predetermined region of the surface, to adhere at least a part of a leaf (8) of a plant to an sealing layer (12), thereby forming a space (6d) between the film (11) and the leaf (8) in the above-mentioned predetermined region of the film (11); a step of measuring the concentration of at least one of water vapor and carbon dioxide inside and outside the space (6d); and a step of calculating at least one of the transpiration rate and the carbon dioxide assimilation rate in a leaf (8) of a plant based on the concentration difference measured between the inside and outside of the space (6d) of at least one of water vapor and carbon dioxide and the above-mentioned permeation resistance.

In the method for measuring biological information of the present embodiment, the sensor (3), the permeable membrane (5), the space (6d), the plant (7), the film (11), the sealing layer (12), and the calculation of the biological information of the plant are as described above and will not be described in detail. In addition, in the method for measuring biological information of the present embodiment, the biological information measuring device (1) including the above-mentioned film (11) can be suitably used.

The method for measuring biological information according to the present embodiment may further include the following steps.

A step of further covering the back side of a leaf (8) of a plant covered by the film (11) with a gas barrier membrane (10), or a step of using the other film (11) constituted by a permeable membrane (5) having permeation resistance to water vapor or carbon dioxide and an sealing layer (12) provided on the surface of the permeable membrane (5) so as to surround a predetermined region of the surface, to adhere the back side of a leaf (8) of a plant to the sealing layer (12) of the other film (11), thereby further forming a space between the other film (11) and the back side of the leaf (8) in the above-mentioned predetermined region of the other film (11).

In this way, it is possible to measure biological information of plants even if the leaf (8) of the plants is two-sided stomatal.

The measurement of the concentration of at least one of water vapor and carbon dioxide in the air outside the space (6d) is not limited to the method of measurement using a sensor (3) provided outside a predetermined region on the surface of the permeable membrane (5), but may be performed, for example, by installing an outer sensor (3) at a position away from the film (11). In addition, when measuring biological information on multiple plant bodies simultaneously, one outer sensor (3) may be used for multiple films (11), or one outer sensor (3) may be used for all films (11).

According to the method for measuring biological information of a plant of the present embodiment, a space (6d) is formed between the film (11) and the leaf (8) by adhering the sealing layer (12) provided on the surface of the permeable membrane (5) in the film so as to surround a predetermined region of the surface to a leaf (8) of a plant, and the concentration of at least one of water vapor and carbon dioxide inside and outside the space (6d) can be measured. The concentration of at least one of water vapor and carbon dioxide measured in this manner can be used to calculate the biological information of the plant body from the permeation resistance of the permeable membrane (5) to water vapor or carbon dioxide. Therefore, in the method for measuring biological information of the present embodiment, it is not necessary to obtain the flow rate of air in the space (6d) or to use a gas flow control device to measure the biological information. In addition, since the air inside the space (6d) is open to the outside air through the permeable membrane (5), there is no risk of the water vapor in the air in the space (6d) becoming saturated or the carbon dioxide becoming depleted. Therefore, the method for measuring biological information according to the present embodiment can measure biological information easily and continuously.

EXAMPLES

Examples are given below to explain in detail the biological information measuring device and biological information measuring method according to the present invention. Specifically, in these examples, a measuring device was fabricated as described below so that the conventional ventilation type measuring method and the measuring method according to the present invention can be simultaneously evaluated and compared.

(Material)

The materials used in the preparation of the biological information measuring device are as follows: A processed acrylic plate (self-prepared) was used as the container, and the following six types of gas permeable membranes were used: nylon mesh 508/585 (mesh size 5 $\mu$m, opening ratio 2%, thread diameter 30/38 $\mu$m), nylon mesh 508/560 (mesh size 10 $\mu$m, opening ratio 4%, thread diameter 30/38 $\mu$m), nylon mesh 520/560 (mesh size 12 $\mu$m, opening ratio 11%, thread diameter 30 $\mu$m), nylon mesh 508 (mesh size 20 $\mu$m, opening ratio 16%, thread diameter 30 $\mu$m), nylon mesh 330 (mesh size 40 $\mu$m, opening ratio 26%, thread diameter 38 $\mu$m), and nylon mesh 254 (mesh size 70 $\mu$m, opening ratio 49%, thread diameter 30 $\mu$m). The blower pump used was a Microblower MZB1001T02 (manufactured by Murata Manufacturing Co., Ltd.), and the gas barrier membrane used was a Propafilm (manufactured by INNOVIA).

(Biological information measuring device)

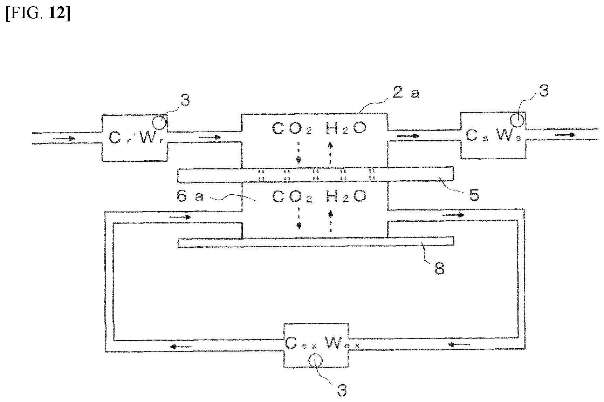

A biological information measuring device was produced, which was equipped with a processed acrylic plate as a container, an LI-7000 as a gas analyzer, at least a part of the acrylic wall constituting the container being constituted by a permeable membrane that is any of the nylon meshes described above, and further equipped with a microblower MZB1001T02 as a blower pump. In addition, an LI-6800 (manufactured by LI-COR) was used as a ventilated gas exchange measuring device, and the biological information measuring device and the ventilated gas exchange measuring device were assembled into a single device to produce a biological information measuring device. A schematic view of the biological information measuring device is shown in FIG. 12.

(Permeation resistance of permeable membrane)

The diffusion conductance ($g_{fw}$) of the nylon mesh used as the permeable membrane to water vapor and the diffusion conductance ($g_{fc}$) of the permeable membrane to carbon dioxide were calibrated for each example. And the details will be described in detail in each example.

(Example 1-1)

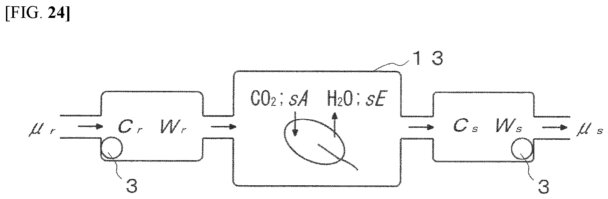

Using the above biological information measuring device, the transpiration rate (E) and carbon dioxide assimilation rate (A) of a leaf of a plant were measured as follows. First, grape, lemon, and cherry were used as plants. All of these plant leaves have stomata only on the abaxial surface. Next, a part of the abaxial surface of the grape leaf, lemon leaf, and cherry leaf was covered with the measurement port of the biological information measuring device, and LI-6800-02 (manufactured by LI-COR), which is an accessory of the LI-6800 chamber, was placed as a light source so as to overlap with the top side of the biological information measuring device. Note that, as the gas permeable membrane, nylon mesh 520/560 was used for the grape leaf and lemon leaf, and nylon mesh 508/585 was used for the cherry leaf. In addition, the surface of the above leaf was covered with a container equipped with a leaf temperature sensor. Next, while keeping $C_r$ fixed at 500 $\mu$molmol$^{-1}$, the LED (photosynthetically active radiation (hereinafter also referred to as "PAR ($\mu$molm$^{-2}$s$^{-1}$)" by mixing two wavelengths of blue (453 nm peak) and red (660 nm peak)) was changed using LI-6800-02, and the inlet water vapor concentration ($W_r$) and outlet water vapor concentration ($W_s$) of the ventilation-type biological information measuring device, as well as the inlet carbon dioxide concentration ($C_r$) and outlet carbon dioxide concentration ($C_s$) were measured, and the inlet air flow rate ($\mu_r$) was measured, and the transpiration rate (hereinafter also referred to as "$E_{STD}$") and the carbon dioxide assimilation rate (hereinafter also referred to as "$A_{STD}$") measured by the ventilation-type measuring device were calculated based on the following formulas (14) and (15) (see the model shown in FIG. 24).

[Mathematical Formula 9]

$$E_{STD} = \frac{\mu_r(W_s - W_r)}{s_l(1 - W_s)} \qquad (14)$$

$$A_{STD} = \frac{\mu_r\left[C_r - C_s\left(\frac{1 - W_r}{1 - W_s}\right)\right]}{s_l} \qquad (15)$$

In formulas (14) and (15), $S_l$ represents the leaf area.

In the present embodiment, $S_l$ represents the area of the leaf covered by the measurement port, and $S_l = 9$ cm$^2$.

Using $E_{STD}$ and $A_{STD}$ measured when PAR = 800 $\mu$molm$^{-2}$s$^{-1}$, the diffusion conductance ($g_{fw}$) of the permeable membrane for water vapor and the diffusion conductance ($g_{fc}$) for carbon dioxide were estimated based on the following formulas (16) and (17) in accordance with the relationships in formulas (12) and (13).

[Mathematical Formula 10]

$$g_{fw} = \frac{W_{ex} - W_a}{E_{STD}} \qquad (16)$$

$$g_{fc} = \frac{C_a - C_{ex}}{A_{STD}} \qquad (17)$$

Simultaneously with the above ventilation-type measurements, the measurement method of the present invention was used to measure the water vapor concentration difference ($W_{ex} - W_a$) and carbon dioxide concentration difference ($C_a - C_{ex}$) between the inside and outside of the space formed by the container and the leaf in the biological information measuring device, and the transpiration rate (hereinafter also referred to as "$E_{NEW}$") and the carbon dioxide assimilation rate (hereinafter also referred to as "$A_{NEW}$") measured by the measurement of the present invention were calculated based on the above formulas (12) and (13) using the diffusion conductance for water vapor ($g_{fw}$) and the diffusion conductance for carbon dioxide ($g_{fc}$) calibrated under the conditions of PAR = 800 $\mu$molm$^{-2}$s$^{-1}$ and $C_r = 500$ $\mu$molmol$^{-1}$ as described above.

Next, while keeping PAR fixed at 800 $\mu$molm$^{-2}$s$^{-1}$, $C_r$ was controlled to change the carbon dioxide concentration in the container (hereinafter also referred to as "$C_s$ ($\mu$molmol$^{-1}$)"), and the transpiration rate ($E_{STD}$) and the carbon dioxide assimilation rate ($A_{STD}$) measured by the ventilation method were obtained as described above. In addition, using $E_{STD}$ and $A_{STD}$ measured when $C_r = 500$ $\mu$molmol$^{-1}$, the diffusion conductance ($g_{fw}$) of the permeable membrane to water vapor and the diffusion conductance ($g_{fc}$) to carbon dioxide were estimated based on the above formulas (16) and (17).

The difference in water vapor concentration ($W_{ex} - W_a$) and the difference in carbon dioxide concentration ($C_a - C_{ex}$) between the inside and outside of the space formed by the container and the leaf in the biological information measuring device were measured, and the transpiration rate ($E_{NEW}$) and the carbon dioxide assimilation rate ($A_{NEW}$) by the measurements according to the present invention were calculated using the diffusion conductance for water vapor ($g_{fw}$) and the diffusion conductance for carbon dioxide ($g_{fc}$) calibrated under the conditions of PAR = 800 $\mu$molm$^{-2}$s$^{-1}$ and $C_r = 500$ $\mu$molmol$^{-1}$ as described above.

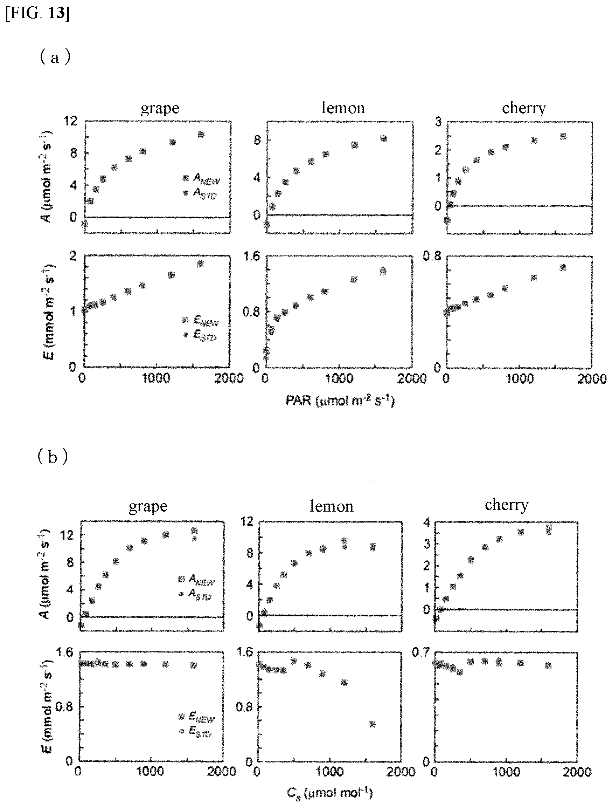

The measurement results are shown in FIG. 13, where FIG. 13(a) shows the light responsiveness of the transpiration rate ($E_{NEW}$, $E_{STD}$) and carbon dioxide assimilation rate ($A_{NEW}$, $A_{STD}$) of grape leaf, lemon leaf, and cherry leaf, and FIG. 13(b) shows the carbon dioxide responsiveness of the transpiration rate ($E_{NEW}$, $E_{STD}$) and carbon dioxide assimilation rate ($A_{NEW}$, $A_{STD}$) of grape leaf, lemon leaf, and cherry leaf. In FIGS. 13(a) and (b), the results ($E_{NEW}$, $A_{NEW}$) obtained by the measurement method according to the present invention are indicated by ■, and the results ($E_{STD}$, $A_{STD}$) obtained by the conventional ventilation type measurement method are indicated by ●.

(Example 1-2)

Using the above biological information measuring device, the transpiration rate (E) and carbon dioxide assimilation rate (A) of a leaf of a plant were measured as follows. First, grapes were used as the plant. As described above, grape leaf is one-sided stomatal. Next, a leaf temperature sensor was attached to the abaxial surface of the grape leaf, and a part of the abaxial surface of the grape leaf containing the leaf temperature sensor was covered with the measurement port of the biological information measuring device. In addition, LI-6800-02 (manufactured by LI-COR), an accessory of the LI-6800 chamber, was placed as a light source on the surface of the grape leaf. Note that nylon mesh 520/560 was used as the gas permeable membrane. Next, while keeping $C_r = 500$ $\mu$molmol$^{-1}$ fixed, LI-6800-02 was used to change the PAR ($\mu$molm$^{-2}$s$^{-1}$) by a two-wavelength mixed LED of blue (453 nm peak) and red (660 nm peak), and the water vapor concentration ($W_r$) at the inlet and the water vapor concentration ($W_s$) at the outlet of the ventilation type biological information measuring device, and the carbon dioxide concentration ($C_r$) at the inlet and the carbon dioxide concentration ($C_s$) at the outlet were measured, as well as the inlet air flow rate ($\mu_r$), and the transpiration rate ($E_{STD}$) and the carbon dioxide assimilation rate ($A_{STD}$) by the ventilation type measurement were obtained based on the above formulas (14) and (15). In this example, $S_l$ represents the area of the leaf covered by the measurement port, and $S_l = 9$ cm$^2$. In addition, the diffusion conductance ($g_{fw}$) of the permeable membrane for water vapor and the diffusion conductance ($g_{fc}$) for carbon dioxide were estimated from the $E_{STD}$ and $A_{STD}$ measured when PAR = 800 $\mu$molm$^{-2}$s$^{-1}$ based on the above formulas (16) and (17).

Simultaneously with the above ventilation-type measurements, the measurement method of the present invention was used to measure the water vapor concentration difference ($W_{ex} - W_a$) and carbon dioxide concentration difference ($C_a - C_{ex}$) between the inside and outside of the space formed by the container and the leaf in the biological information measuring device, and the transpiration rate ($E_{NEW}$) and carbon dioxide assimilation rate ($A_{NEW}$) by the measurements of the present invention were calculated based on the above formulas (12) and (13) using the diffusion conductance for water vapor ($g_{fw}$) and the diffusion conductance for carbon dioxide ($g_{fc}$) calibrated under the conditions of PAR = 800 $\mu$molm$^{-2}$s$^{-1}$ and $C_r = 500$ $\mu$molmol$^{-1}$ as described above.

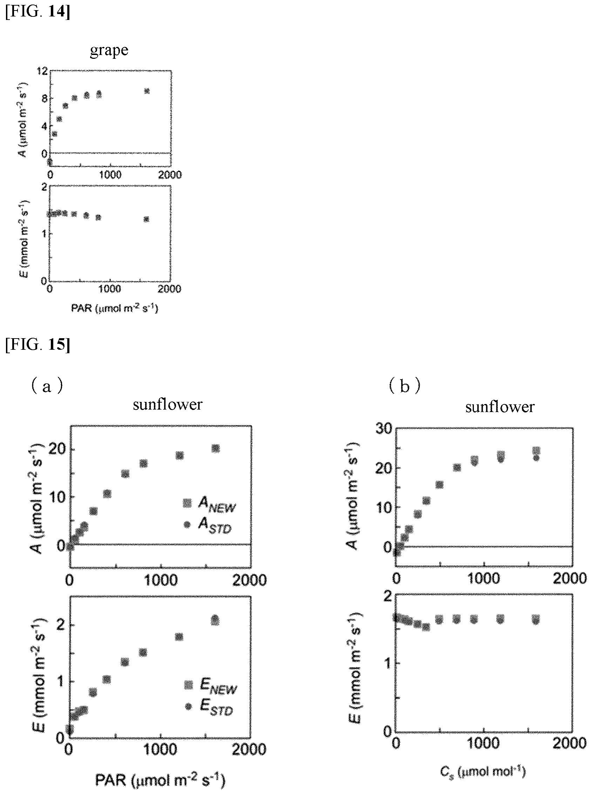

The measurement results are shown in FIG. 14, which shows the light responsiveness of the transpiration rate ($E_{NEW}$, $E_{STD}$) and carbon dioxide assimilation rate ($A_{NEW}$, $A_{STD}$) of grapevine leaf. In FIG. 14, the results ($E_{NEW}$, $A_{NEW}$) obtained by the measurement method of the present invention are shown by ■, and the results ($E_{STD}$, $A_{STD}$) obtained by the conventional ventilation type measurement method are shown by ●.

(Examples 1 to 3)

Using the above biological information measuring device, the transpiration rate (E) and carbon dioxide assimilation rate (A) of a leaf of a plant were measured as follows. First, a sunflower was used as the plant. Sunflower leaf is two-sided stomatal. Next, a part of the surface of the sunflower leaf was covered with the measurement port of the biological information measuring device, and further, LI-6800-02 (manufactured by LI-COR), an accessory of the LI-6800 chamber, was placed as a light source so as to overlap the top side of the biological information measuring device. Note that nylon mesh 520/560 was used as the gas permeable membrane. In addition, the abaxial surface of the leaf was covered with a container equipped with a leaf temperature sensor. Next, while keeping $C_r = 500$ $\mu$molmol$^{-1}$ fixed, LI-6800-02 was used to change the PAR ($\mu$molm$^{-2}$s$^{-1}$) by the LED of two wavelengths mixed blue (453 nm peak) and red (660 nm peak), and the water vapor concentration ($W_r$) at the inlet and the water vapor concentration ($W_s$) at the outlet of the ventilation type biological information measuring device, and the carbon dioxide concentration ($C_r$) at the inlet and the carbon dioxide concentration ($C_s$) at the outlet were measured, and the inlet air flow rate ($\mu_r$) was measured, and the transpiration rate ($E_{STD}$) and the carbon dioxide assimilation rate ($A_{STD}$) by the ventilation type measurement were obtained based on the above formulas (14) and (15). In this example, $S_l$ represents the area of the leaf covered by the measurement port, and $S_l = 9$ cm$^2$. In addition, the diffusion conductance ($g_{fw}$) of the permeable membrane for water vapor and the diffusion conductance ($g_{fc}$) for carbon dioxide were estimated from the $E_{STD}$ and $A_{STD}$ measured when PAR = 800 $\mu$molm$^{-2}$s$^{-1}$ based on the above formulas (16) and (17).

Simultaneously with the above ventilation-type measurements, the measurement method of the present invention was used to measure the water vapor concentration difference ($W_{ex} - W_a$) and carbon dioxide concentration difference ($C_a - C_{ex}$) between the inside and outside of the space formed by the container and leaf in the biological information measuring device, and the transpiration rate ($E_{NEW}$) and carbon dioxide assimilation rate ($A_{NEW}$) by the measurements of the present invention were calculated based on the above formulas (12) and (13) using the diffusion conductance for water vapor ($g_{fw}$) and the diffusion conductance for carbon dioxide ($g_{fc}$) calibrated under the conditions of

PAR = 800 $\mu$molm$^{-2}$s$^{-1}$ and C$_r$ = 500 $\mu$molmol$^{-1}$ as described above.

Next, while keeping PAR fixed at 800 $\mu$molm$^{-2}$s$^{-1}$, C$_r$ was controlled to change the carbon dioxide concentration in the container (Cs ($\mu$molmol$^{-1}$)), and the transpiration rate (E$_{STD}$) and carbon dioxide assimilation rate (A$_{STD}$) measured by ventilation were determined as described above. In addition, from E$_{STD}$ and A$_{STD}$ measured when C$_r$ = 500 $\mu$molmol$^{-1}$, the diffusion conductance of the permeable membrane to water vapor (g$_{fw}$) and the diffusion conductance to carbon dioxide (g$_{fc}$) were estimated based on the above formulas (16) and (17).

The difference in water vapor concentration (W$_{ex}$ - W$_a$) and the difference in carbon dioxide concentration (C$_a$ - C$_{ex}$) between the inside and outside of the space formed by the container and the leaf in the biological information measuring device were measured, and the transpiration rate (E$_{NEW}$) and the carbon dioxide assimilation rate (A$_{NEW}$) by the measurements according to the present invention were calculated based on the above formulas (12) and (13) using the diffusion conductance for water vapor (g$_{fw}$) and the diffusion conductance for carbon dioxide (g$_{fc}$) calibrated under the conditions of PAR = 800 $\mu$molm$^{-2}$s$^{-1}$ and C$_r$ = 500 $\mu$molmol$^{-1}$ as described above.

The measurement results are shown in FIG. 15, where FIG. 15(a) shows the light responsiveness of the transpiration rate (E$_{NEW}$, E$_{STD}$) and carbon dioxide assimilation rate (A$_{NEW}$, A$_{STD}$) of sunflower leaf, and FIG. 15(b) shows the carbon dioxide responsiveness of the transpiration rate (E$_{NEW}$, E$_{STD}$) and carbon dioxide assimilation rate (A$_{NEW}$, A$_{STD}$) of sunflower leaf. In FIG. 15, the results (E$_{NEW}$, A$_{NEW}$) obtained by the measurement method according to the present invention are indicated by ■, and the results (E$_{STD}$, A$_{STD}$) obtained by the conventional ventilation type measurement method are indicated by ●.

(Examples 1 to 4)

Using the above biological information measuring device, the transpiration rate (E) and carbon dioxide assimilation rate (A) of a leaf of a plant were measured as follows. First, strawberry and sunflower were used as plants. Strawberry leaf is two-sided stomatal, and sunflower leaf is also two-sided stomatal as described above. Next, a leaf temperature sensor was attached to the abaxial surface of the leaf, and a part of the abaxial surface of the strawberry leaf and sunflower leaf containing the leaf temperature sensor was covered by the measurement port of the biological information measuring device. In addition, LI-6800-02 (manufactured by LI-COR), which is an accessory of the LI-6800 chamber, was placed on the surface of the leaf as a light source. Note that, as the gas permeable membrane, nylon mesh 520/560 was used for strawberry leaf, and nylon mesh 508 was used for sunflowers. Next, while keeping C$_r$ = 500 $\mu$molmol$^{-1}$ fixed, LI-6800-02 was used to change the PAR ($\mu$molm$^{-2}$s$^{-1}$) by a two-wavelength mixed LED of blue (453 nm peak) and red (660 nm peak), and the water vapor concentration (W$_r$) at the inlet and the water vapor concentration (W$_s$) at the outlet of the ventilation type biological information measuring device, and the carbon dioxide concentration (C$_r$) at the inlet and the carbon dioxide concentration (C$_s$) at the outlet were measured, as well as the inlet air flow rate ($\mu_r$), and the transpiration rate (E$_{STD}$) and the carbon dioxide assimilation rate (A$_{STD}$) by the ventilation type measurement were obtained based on the above formulas (14) and (15). In this example, S$_l$ represents the area of the leaf covered by the measurement port, and S$_l$ = 9 cm$^2$. In addition, the diffusion conductance (g$_{fw}$) of the permeable membrane for water vapor and the diffusion conductance (g$_{fc}$) for carbon dioxide were estimated from the E$_{STD}$ and A$_{STD}$ measured when PAR = 800 $\mu$molm$^{-2}$s$^{-1}$ based on the above formulas (16) and (17).

Simultaneously with the above ventilation-type measurements, the measurement method of the present invention was used to measure the water vapor concentration difference (W$_{ex}$ - W$_a$) and carbon dioxide concentration difference (C$_a$ - C$_{ex}$) between the inside and outside of the space formed by the container and the leaf in the biological information measuring device, and the transpiration rate (E$_{NEW}$) and carbon dioxide assimilation rate (A$_{NEW}$) by the measurements of the present invention were calculated based on the above formulas (12) and (13) using the diffusion conductance for water vapor (g$_{fw}$) and the diffusion conductance for carbon dioxide (g$_{fc}$) calibrated under the conditions of PAR = 800 $\mu$molm$^{-2}$s$^{-1}$ and C$_r$ = 500 $\mu$molmol$^{-1}$ as described above.

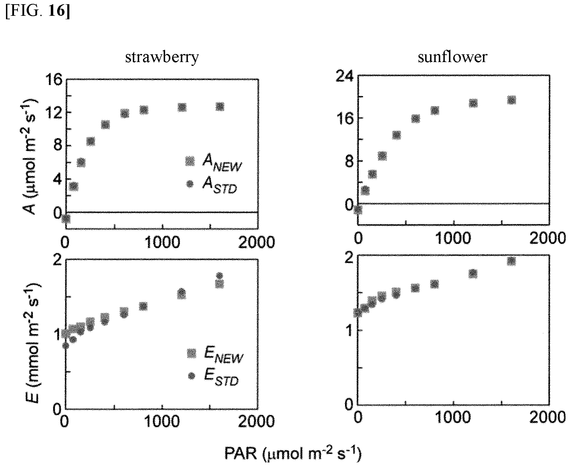

The measurement results are shown in FIG. 16, which shows the light responsiveness of the transpiration rate (E$_{NEW}$, E$_{STD}$) and carbon dioxide assimilation rate (A$_{NEW}$, A$_{STD}$) of strawberry leaf and sunflower leaf. In FIG. 16, the results (E$_{NEW}$, A$_{NEW}$) obtained by the measurement method of the present invention are shown by ■, and the results (E$_{STD}$, A$_{STD}$) obtained by the conventional ventilation type measurement method are shown by ●.

Example 2

The transpiration rate (E) and carbon dioxide assimilation rate (A) of sunflower leaf were measured as follows using permeable membranes with different mesh numbers in the above-mentioned biological information measuring device. The permeable membranes used were nylon mesh 520/260, nylon mesh 508, nylon mesh 330, and nylon mesh 254. Then, a part of the abaxial surface of the sunflower leaf, which is two-sided stomatal, was covered with the measurement port of each biological information measuring device. In addition, LI-6800-02 (manufactured by LI-COR), an accessory of the LI-6800 chamber, was placed on the surface of the sunflower leaf as a light source. Next,

while keeping $C_r$ = 500 $\mu$molmol$^{-1}$ fixed, LI-6800-02 was used to change the PAR ($\mu$molm$^{-2}$s$^{-1}$) by a two-wavelength mixed LED of blue (453 nm peak) and red (660 nm peak), and the water vapor concentration ($W_r$) at the inlet and the water vapor concentration ($W_s$) at the outlet of the ventilation type biological information measuring device, and the carbon dioxide concentration ($C_r$) at the inlet and the carbon dioxide concentration ($C_s$) at the outlet were measured, as well as the inlet air flow rate ($\mu_r$), and the transpiration rate ($E_{STD}$) and the carbon dioxide assimilation rate ($A_{STD}$) by the ventilation type measurement were obtained based on the above formulas (14) and (15). In this example, $S_l$ represents the area of the leaf covered by the measurement port, and $S_l$ = 9 cm$^2$. In addition, the diffusion conductance ($g_{fw}$) of the permeable membrane for water vapor and the diffusion conductance ($g_{fc}$) for carbon dioxide were estimated from the $E_{STD}$ and $A_{STD}$ measured when PAR = 800 $\mu$molm$^{-2}$s$^{-1}$ based on the above formulas (16) and (17).

Simultaneously with the above ventilation-type measurements, the measurement method of the present invention was used to measure the water vapor concentration difference ($W_{ex}$ - $W_a$) and carbon dioxide concentration difference ($C_a$ - $C_{ex}$) between the inside and outside of the space formed by the container and the leaf in the biological information measuring device, and the transpiration rate ($E_{NEW}$) and carbon dioxide assimilation rate ($A_{NEW}$) by the measurements of the present invention were calculated based on the above formulas (12) and (13) using the diffusion conductance for water vapor ($g_{fw}$) and the diffusion conductance for carbon dioxide ($g_{fc}$) calibrated under the conditions of PAR = 800 $\mu$molm$^{-2}$s$^{-1}$ and $C_r$ = 500 $\mu$molmol$^{-1}$ as described above.

Next, while keeping PAR fixed at 800 $\mu$molm$^{-2}$s$^{-1}$, $C_r$ was controlled to change the carbon dioxide concentration in the container (Cs ($\mu$molmol$^{-1}$)), and the transpiration rate ($E_{STD}$) and carbon dioxide assimilation rate ($A_{STD}$) measured by ventilation were determined as described above. In addition, from $E_{STD}$ and $A_{STD}$ measured when $C_r$ = 500 $\mu$molmol$^{-1}$, the diffusion conductance of the permeable membrane to water vapor ($g_{fw}$) and the diffusion conductance to carbon dioxide ($g_{fc}$) were estimated based on the above formulas (16) and (17).

The difference in water vapor concentration ($W_{ex}$ - $W_a$) and the difference in carbon dioxide concentration ($C_a$ - $C_{ex}$) between the inside and outside of the space formed by the container and the leaf in the biological information measuring device were measured, and the transpiration rate ($E_{NEW}$) and the carbon dioxide assimilation rate ($A_{NEW}$) by the measurements according to the present invention were calculated based on the above formulas (12) and (13) using the diffusion conductance for water vapor ($g_{fw}$) and the diffusion conductance for carbon dioxide ($g_{fc}$) calibrated under the conditions of PAR = 800 $\mu$molm$^{-2}$s$^{-1}$ and $C_r$ = 500 $\mu$molmol$^{-1}$ as described above.

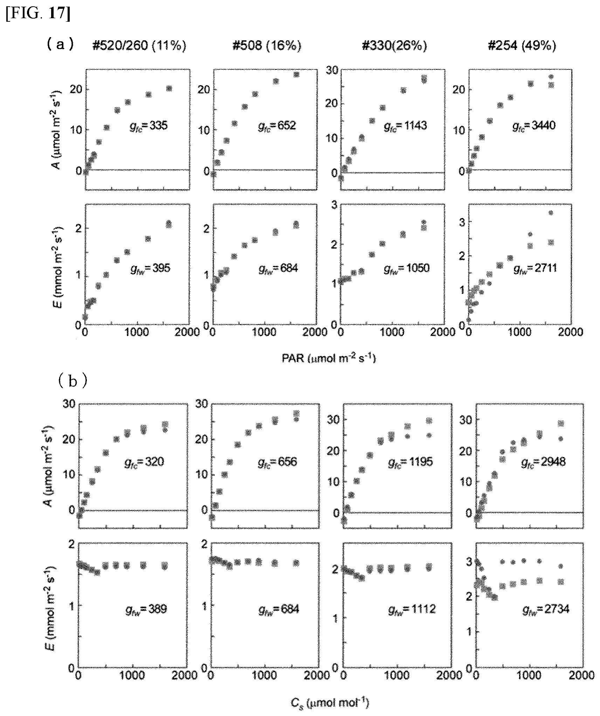

The measurement results are shown in FIG. 17, where FIG. 17(a) shows the light responsiveness of the transpiration rate ($E_{NEW}$, $E_{STD}$) and carbon dioxide assimilation rate ($A_{NEW}$, $A_{STD}$) of sunflower leaf when permeable membranes with different mesh numbers are used, and FIG. 17(b) shows the carbon dioxide responsiveness of the transpiration rate ($E_{NEW}$, $E_{STD}$) and carbon dioxide assimilation rate ($A_{NEW}$, $A_{STD}$) of sunflower leaf when permeable membranes with different mesh numbers are used. In FIG. 17, # indicates the mesh number, and the numbers in parentheses indicate the opening rate. In FIG. 17, the results ($E_{NEW}$, $A_{NEW}$) obtained by the measurement method of the present invention are indicated by ■, and the results ($E_{STD}$, $A_{STD}$) obtained by the conventional ventilation type measurement method are indicated by ●.

Example 3

Using the above biological information measuring device, the transpiration rate (E) and carbon dioxide assimilation rate (A) of sunflower leaf were measured under different temperature conditions as follows. First, a part of the surface of a sunflower leaf was covered with the measurement port of the biological information measuring device, and further, LI-6800-02 (manufactured by LI-COR), an accessory of the LI-6800 chamber, was placed as a light source so as to overlap the top side of the biological information measuring device. In addition, the abaxial surface of the leaf was covered with a container equipped with a leaf temperature sensor. Next, while fixing the photosynthetically active radiation (PAR) = 800 $\mu$molm$^{-2}$s$^{-1}$ and $C_r$ = 500 $\mu$molmol$^{-1}$ using a two-wavelength mixed LED of blue (453 nm peak) and red (660 nm peak) using the LI-6800-02, and controlling the temperature of the air supplied to the chamber of the LI-6800 (hereinafter also referred to as "$T_{air}$ (°C)") to change the leaf temperature (hereinafter also referred to as "$T_{leaf}$ (°C)"), the inlet water vapor concentration ($W_r$) and outlet water vapor concentration ($W_s$) of the ventilation-type biological information measuring device, the inlet carbon dioxide concentration ($C_r$) and outlet carbon dioxide concentration ($C_s$) were measured, and the inlet air flow rate ($\mu_r$) was measured, and the transpiration rate ($E_{STD}$) and carbon dioxide assimilation rate ($A_{STD}$) measured by the ventilation-type measurement were obtained based on the above formulas (14) and (15). In addition, the diffusion conductance ($g_{fw}$) of the permeable membrane for water vapor and the diffusion conductance ($g_{fc}$) for carbon dioxide were measured based on the above formulas (16) and (17) from the $E_{STD}$ and $A_{STD}$ measured when $T_{air}$ = 18°C. This measurement was repeated three times for each $T_{air}$. In this example, $S_l$ represents the area of the leaf covered by the measurement port, and $S_l$ = 9 cm$^2$.

In addition, the carbon dioxide concentration difference ($C_a$ - $C_{ex}$) between the inside and outside of the space formed by the container and the leaf in the biological information measuring device was measured while changing the leaf temperature ($T_{leaf}$). Using the diffusion conductance ($g_{fc}$) for carbon dioxide calibrated under the conditions of PAR = 800

$\mu$molm$^{-2}$s$^{-1}$, $C_r$ = 500 $\mu$molmol$^{-1}$ and $T_{air}$ = 18°C measured as described above, the carbon dioxide assimilation rate ($A_{NEW}$) by the measurement according to the present invention was calculated based on the above formula (13).

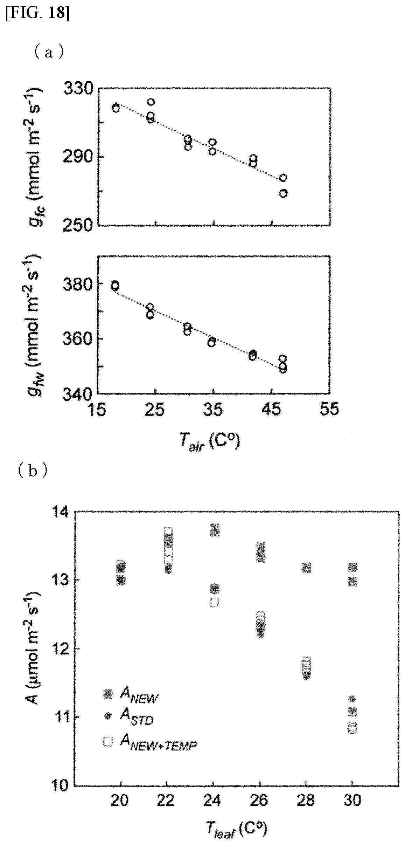

The measurement results are shown in FIG. 18, where FIG. 18(a) shows the diffusion conductance ($g_{fw}$) of the permeable membrane for water vapor and the diffusion conductance ($g_{fc}$) for carbon dioxide in $T_{air}$, and FIG. 18(b) shows the carbon dioxide assimilation rate in $T_{leaf}$ ($A_{NEW}$, $A_{STD}$, $A_{NEW + TEMP}$). In FIG. 18, $A_{NEW + TEMP}$ shows $A_{NEW}$ when $g_{fc}$ is temperature corrected using the linear regression equation shown in FIG. 18(a). In FIG. 18(b), the results ($A_{NEW}$) obtained by the measurement method according to the present invention are shown by ■, the results ($A_{STD}$) obtained by the conventional ventilation type measurement method are shown **by ●,** and the temperature corrected results ($A_{NEW + TEMP}$) are shown by □.

Example 4

Using the above-mentioned biological information measuring device, whether a decrease in the carbon dioxide assimilation rate (A) associated with aging of sunflower leaf can be detected was verified as follows. Note that nylon mesh 330 was used as the permeable membrane. First, a part of the surface of each sunflower leaf was covered with the measurement port of the biological information measuring device, and further, LI-6800-02 (manufactured by LI-COR), an accessory of the LI-6800 chamber, was placed as a light source so as to overlap the top side of the biological information measuring device. Then, using LI-6800-02, photosynthetically active radiation (PAR) was fixed at 1600 $\mu$molm$^{-2}$s$^{-1}$ and $C_r$ = 500 $\mu$molmol$^{-1}$ using a two-wavelength mixed LED of blue (453 nm peak) and red (660 nm peak), and the water vapor concentration ($W_r$) at the inlet and the water vapor concentration ($W_s$) at the outlet of the ventilation-type biological information measuring device, as well as the carbon dioxide concentration ($C_r$) at the inlet and the carbon dioxide concentration ($C_s$) at the outlet were measured, and the inlet air flow rate ($\mu_r$) was measured, and the carbon dioxide assimilation rate ($A_{STD}$) by the ventilation-type measurement was calculated based on the above formula (15). This measurement was performed from the new leaf (leaf position = 1) located at the top of the sunflower to the old leaf (leaf position = 10) located at the bottom. In the present embodiment, $S_l$ represents the area of the leaf covered by the measurement pore, and $S_l$ =9 cm$^2$.

The carbon dioxide concentration difference ($C_a$ - $C_{ex}$) between the inside and outside of the space formed by the container and the leaf in the biological information measuring device was measured under the condition of PAR = 1600 $\mu$molm$^{-2}$s$^{-1}$ using a 2-wavelength mixed LED of blue (453 nm peak) and red (660 nm peak) using LI-6800-02, and the carbon dioxide assimilation rate ($A_{NEW}$) by the measurement according to the present invention was calculated based on the above formula (13) using the diffusion conductance ($g_{fc}$) of nylon mesh 330 used as a permeable membrane to carbon dioxide. Note that for $g_{fc}$ of nylon mesh 330, a value calibrated under the conditions of PAR = 800 $\mu$molm$^{-2}$s$^{-1}$ and $C_r$ = 500 $\mu$molmol$^{-1}$ in Example 2 was used.

Furthermore, for comparison, the SPAD value of each sunflower leaf was measured using a chlorophyll meter SPAD-502Plus (manufactured by Konica Minolta).

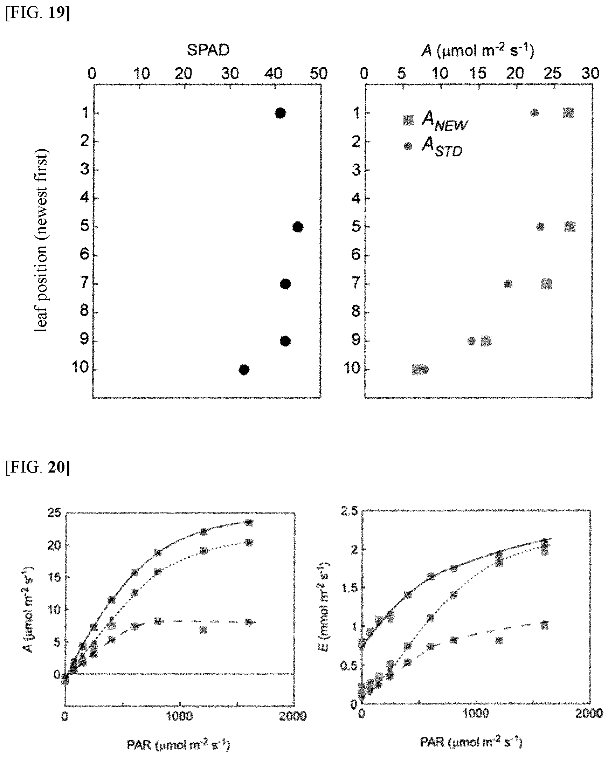

The measurement results are shown in FIG. 19, where the left side of FIG. 19 shows the SPAD value at the leaf position, and the right side of FIG. 19 shows the carbon dioxide assimilation rate ($A_{NEW}$, $A_{STD}$) at the leaf position. Note that in the right side of FIG. 19, the results ($A_{NEW}$) obtained by the measurement method according to the present invention are indicated by ■, and the results obtained by the conventional ventilation type measurement method are indicated by ● ($A_{STD}$).

(Example 5-1)

Using the above biological information measuring device, the effects on the transpiration rate (E) and carbon dioxide assimilation rate (A) when sunflower leaf are exposed to water stress were examined as follows. The permeable membrane used was nylon mesh 520/560, and irrigation was stopped as the water stress applied to the sunflower. First, a part of the surface of the sunflower leaf was covered with the measurement port of the biological information measuring device, and further, LI-6800-02 (manufactured by LI-COR), an accessory of the LI-6800 chamber, was placed as a light source so as to overlap the top side of the biological information measuring device. Next, for sunflower leaf before irrigation was stopped, 3 days after irrigation was stopped, and 4 days after irrigation was stopped, the PAR ($\mu$molm$^{-2}$s$^{-1}$) of a two-wavelength mixed LED of blue (peak at 453 nm) and red (peak at 660 nm) was changed using an LI-6800-02 with $C_r$ fixed at 500 $\mu$molmol$^{-1}$, while the inlet water vapor concentration ($W_r$), outlet water vapor concentration ($W_s$), inlet carbon dioxide concentration ($C_r$) and outlet carbon dioxide concentration ($C_s$) of the ventilated biological information measuring device were measured, and the inlet air flow rate ($\mu_r$) was measured, and the transpiration rate ($E_{STD}$) and carbon dioxide assimilation rate ($A_{STD}$) measured by the ventilated measurement were calculated based on the above formula (13). In this example, $S_1$ represents the area of the leaf covered by the measurement port, and $S_1$ = 9 cm$^2$. In addition, the diffusion conductance ($g_{fw}$) of the permeable membrane for water vapor and the diffusion conductance ($g_{fc}$) for carbon dioxide were estimated from $E_{STD}$ and $A_{STD}$ measured when PAR = 800 $\mu$molm$^{-2}$s$^{-1}$, based on the above formulas (16) and (17).

In addition, the water vapor concentration difference ($W_{ex} - W_a$) and carbon dioxide concentration difference ($C_a - C_{ex}$) between the inside and outside of the space formed by the container and the leaf in the biological information measuring device were measured while changing the PAR ($\mu molm^{-2}s^{-1}$) of a two-wavelength mixed LED of blue (453 nm peak) and red (660 nm peak) using LI-6800-02. Using the diffusion conductance for water vapor ($g_{fw}$) and the diffusion conductance for carbon dioxide ($g_{fc}$) calibrated under the conditions of PAR = 800 $\mu molm^{-2}s^{-1}$ and $C_r$ = 500 $\mu molmol^{-1}$ as described above, the transpiration rate ($E_{NEW}$) and the carbon dioxide assimilation rate ($A_{NEW}$) by the measurement according to the present invention were calculated based on the above formulas (12) and (13).

The measurement results are shown in FIG. 20, where the upper part of FIG. 20 shows the light responsiveness of the carbon dioxide assimilation rate ($A_{NEW}$, $A_{STD}$) before irrigation was stopped (solid line), 3 days after irrigation was stopped (dotted line), and 4 days after irrigation was stopped (dashed line), and the lower part of FIG. 20 shows the light responsiveness of the transpiration rate ($E_{NEW}$, $E_{STD}$) before irrigation was stopped (solid line), 3 days after irrigation was stopped (dotted line), and 4 days after irrigation was stopped (dashed line). In FIG. 20, the results of the measurement according to the present invention ($E_{NEW}$, $A_{NEW}$) are indicated by ■, and the results of the conventional ventilation-type measurement method ($E_{STD}$, $A_{STD}$) are indicated by ●.

(Example 5-2)

The above biological information measuring device was used to continuously measure the transpiration rate (E) and carbon dioxide assimilation rate (A) of sunflower leaf, and the effect of diurnal changes when sunflower leaf was exposed to water stress was examined as follows. The permeable membrane was nylon mesh 508, and the water stress on the sunflower was the suspension of irrigation as in Example 5-1. The measurement period was from the suspension of irrigation to 5 days after the suspension of irrigation, and irrigation was performed once at 18:00 on the 4th day. First, a part of the surface of the sunflower leaf was covered with the measurement port of the biological information measuring device, and LI-6800-02 (manufactured by LI-COR), an accessory of the LI-6800 chamber, was placed as a light source so as to overlap with the top side of the biological information measuring device, and the pot containing the sunflower to be measured was placed on the electronic balance UP8201X (manufactured by Shimadzu Corporation). The weight (g) of the pot over time was measured, and the difference in water vapor concentration ($W_{ex} - W_a$) and the difference in carbon dioxide concentration ($C_a - C_{ex}$) between the inside and outside of the space formed by the container and the leaf in the biological information measuring device were measured. Using the diffusion conductance ($g_{fw}$) for water vapor and the diffusion conductance ($g_{fc}$) for carbon dioxide of nylon mesh 508 used as a permeable membrane, the transpiration rate ($E_{NEW}$) and the carbon dioxide assimilation rate ($A_{NEW}$) measured according to the present invention were calculated based on the above formulas (12) and (13). Note that for $g_{fw}$ and $g_{fc}$ of nylon mesh 508, values calibrated under the conditions of PAR=800 $\mu molm^{-2}s^{-1}$ and $C_r$ =500 $\mu molmol^{-1}$ on the day before irrigation was stopped were used. For comparison, the transpiration rate ($E_{NEW}$) and carbon dioxide assimilation rate ($A_{NEW}$) of the leaf of the sunflowers on the day before irrigation was stopped were measured using a permeable membrane and plotted on a graph, and this graph was repeatedly displayed for each date. Note that $E_{NEW}$ and $A_{NEW}$ were calculated in the same manner as above, based on the above formulas (12) and (13).

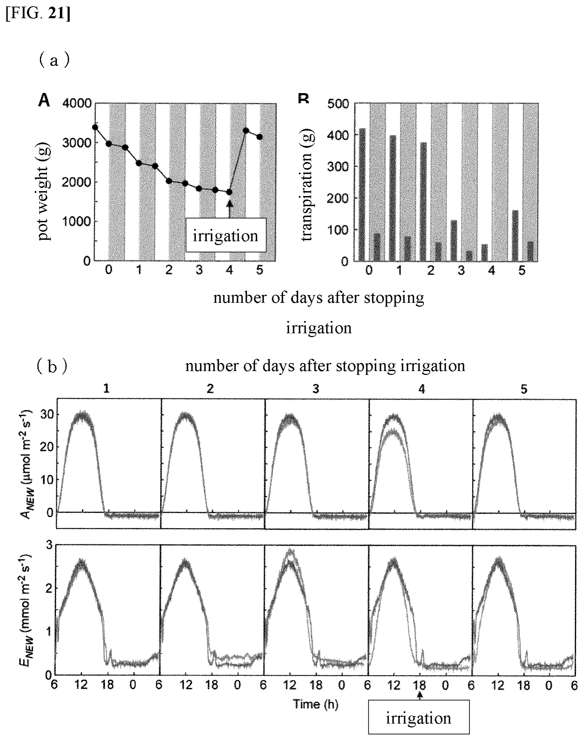

The measurement results are shown in FIG. 21. The left side of FIG. 21(a) shows the change in the weight (g) of the sunflower pot over time, and the right side of FIG. 21(a) shows the change in the amount of evapotranspiration (g) from the sunflower and the soil in the pot over time. FIG. 21(b) shows the change in the transpiration rate ($E_{NEW}$) and the carbon dioxide assimilation rate ($A_{NEW}$) of the sunflower leaf over time. In FIG. 21(a), the areas with a white background indicate the daytime (6:00-17:00), and the areas with a gray background indicate the nighttime (17:00-6:00). FIG. 21(b) shows both the results for the sunflower leaf after irrigation was stopped ($E_{NEW}$, $A_{NEW}$) and the results for the sunflower leaf before irrigation was stopped ($E_{NEW}$, $A_{NEW}$).

(Result)

As shown in FIGS. 13(a) and (b), it was confirmed that when a biological information measuring device using a permeable membrane and a light source were placed on the abaxial surface of grape leaf, lemon leaf, and cherry leaf, which are one-sided stomatal, the transpiration rate (E) and carbon dioxide assimilation rate (A) of each leaf could be measured by the biological information measuring device. Furthermore, as shown in FIG. 13(a), when the measurement results of the biological information measuring device using a permeable membrane and the ventilated LI-6800 were compared, it was confirmed that the measurement results of the light responsiveness at the transpiration rate ($E_{NEW}$, $E_{STD}$) and carbon dioxide assimilation rate ($A_{NEW}$, $A_{STD}$) of each leaf were almost the same, and as shown in FIG. 13(b), it was confirmed that the measurement results of the carbon dioxide responsiveness at the transpiration rate ($E_{NEW}$, $E_{STD}$) and carbon dioxide assimilation rate ($A_{NEW}$, $A_{STD}$) of each leaf were also almost the same.

As shown in FIG. 14, it was confirmed that even when a biological information measuring device using a permeable membrane was placed on the abaxial surface of a grape leaf, which is one-sided stomatal, and a light source was placed on the surface of the grape leaf, the biological information measuring device was able to measure the transpiration rate (E) and carbon dioxide assimilation rate (A) of the grape leaf. Furthermore, as shown in FIG. 14, when the measurement results of the biological information measuring device using a permeable membrane and the ventilated LI-6800 are compared, it was confirmed that the measurement results of the light responsiveness of the transpiration rate ($E_{NEW}$, $E_{STD}$) and carbon dioxide assimilation rate ($A_{NEW}$, $A_{STD}$) of grape leaf are almost the same.

As shown in FIG. 15, when a biological information measuring device using a permeable membrane and a light source were placed on the surface of a sunflower leaf, which is two-sided stomatal, and the abaxial surface of the sunflower leaf was covered with a container, it was confirmed that the transpiration rate (E) and carbon dioxide assimilation rate (A) of the sunflower leaf could be measured by the biological information measuring device.

Furthermore, as shown in FIG. 15(a), when the measurement results of the biological information measuring device using a permeable membrane and the ventilated LI-6800 were compared, it was confirmed that the measurement results of the light responsiveness of the transpiration rate ($E_{NEW}$, $E_{STD}$) and the carbon dioxide assimilation rate ($A_{NEW}$, $A_{STD}$) of each leaf were almost the same, and as shown in FIG. 15(b), it was confirmed that the measurement results of the carbon dioxide responsiveness of the transpiration rate ($E_{NEW}$, $E_{STD}$) and the carbon dioxide assimilation rate ($A_{NEW}$, $A_{STD}$) of each leaf were also almost the same.

It was confirmed that even when a biological information measuring device using a permeable membrane is placed on the abaxial surface of strawberry leaf and sunflower leaf, which are two-sided stomatal, and a gas barrier membrane and a light source are placed on the surface of the leaf, the transpiration rate (E) and the carbon dioxide assimilation rate (A) of strawberry leaf and sunflower leaf can be measured by the biological information measuring device, as shown in FIG. 16. Furthermore, it was confirmed that when the measurement results of the biological information measuring device using a permeable membrane and the ventilated LI-6800 are compared, the measurement results of the light responsiveness of the transpiration rate ($E_{NEW}$, $E_{STD}$) and the carbon dioxide assimilation rate ($A_{NEW}$, $A_{STD}$) of each leaf are almost the same, as shown in FIG. 16, and further, it was confirmed that the measurement results of the carbon dioxide responsiveness of the transpiration rate ($E_{NEW}$, $E_{STD}$) and the carbon dioxide assimilation rate ($A_{NEW}$, $A_{STD}$) of each leaf are also almost the same, as shown in FIG. 16(b).

From the results of FIGS. 13 to 16, it was shown that the biological information measuring device using the permeable membrane can detect the biological information of plants, such as the transpiration rate (E) and the carbon dioxide assimilation rate (A), with accuracy comparable to that of the ventilated LI-6800 (manufactured by LI-COR), which is currently used as the global standard. It was also confirmed that the measurement method according to the present invention does not require obtaining the air flow rate in the container or using a gas flow control device to measure the biological information, and there is no risk of the water vapor in the air in the space becoming saturated or carbon dioxide becoming depleted. From the above results, it was confirmed that the measurement method according to the present invention can easily and continuously measure the biological information of a plant body.

From the results of FIGS. 13 to 16, it was confirmed that when the leaf of a plant is one-sided stomatal, the abaxial surface of the leaf having stomata can be covered with the container of the biological information measuring device using a permeable membrane to measure the biological information of the plant. On the other hand, when the leaf of a plant is two-sided stomatal, it was confirmed that the biological information of the plant can be measured by covering at least one of the adaxial and abaxial side of the leaf with the container of the biological information measuring device using a permeable membrane, and covering the other leaf surface with a gas barrier membrane or with a container. Therefore, it was shown that the biological information measuring device using a permeable membrane can measure the biological information of the plant whether a leaf of a plant is one-sided stomatal or two-sided stomatal.

As shown in FIGS. 17(a) and (b), it was confirmed that when the opening ratio of the permeable membrane in the biological information measuring device is 11% to 49%, the biological information of the plant can be measured with high accuracy. In particular, when the opening ratio of the permeable membrane is 11% to 26%, more accurate measurement is possible. It was confirmed that the smaller the mesh number, that is, the higher the opening ratio, the higher the diffusion conductance ($g_{fc}$, $g_{fw}$), that is, the easier it is for gases such as water vapor or carbon dioxide to permeate. From the results of FIG. 17, it is preferable that the diffusion conductance of the permeable membrane is 3500 mmolm$^{-2}$s$^{-1}$ or less, and more preferably that the diffusion conductance of the permeable membrane to carbon dioxide is 1200 mmolm$^{-2}$s$^{-1}$ or less, and more preferably that the diffusion conductance of the permeable membrane to water vapor is 1100 mmolm$^{-2}$s$^{-1}$ or less.

As shown in FIG. 18(a), it was confirmed that the higher the air temperature, the lower the diffusion conductance ($g_{fw}$) of the permeable membrane for water vapor and the diffusion conductance ($g_{fc}$) for carbon dioxide. In particular, since both $g_{fw}$ and $g_{fc}$ decreased, it is considered that the physical change of the mesh pores, such as the decrease in the opening ratio due to the thermal expansion of the resin fiber, is the cause. In addition, as shown in FIG. 18(b), it was confirmed that temperature correction of the diffusion conductance is necessary to identify the optimal temperature for photosynthesis at which the carbon dioxide assimilation rate (A) is maximized.

As shown in FIG. 19, it was confirmed that the older the leaf, the lower the SPAD value, as measured using a chlorophyll meter SPAD-502Plus (manufactured by Konica Minolta). It was also confirmed that the older the leaf, the lower the carbon dioxide assimilation rate ($A_{NEW}$, $A_{STD}$) as measured using the measurement method according to the present invention and the conventional ventilation method.

As shown in FIG. 20, it was confirmed that the measurement method according to the present invention can measure the transpiration rate (E) and carbon dioxide assimilation rate (A) decreased by water stress with the same level of accuracy as the conventional ventilation method.

As shown in FIG. 21(a), it was confirmed that about 500 g of water was lost from the pot every day until two days after irrigation was stopped. This is presumably mainly due to evaporation during the day. Next, it was confirmed that the amount of evapotranspiration significantly decreased from three days after irrigation was stopped, and the amount of evapotranspiration during the day four days later was about 50 g. On the other hand, when irrigation was resumed at 18:00, which is after sunset on the fourth day after irrigation was stopped, the amount of evapotranspiration during the day the next day was confirmed to recover to about 160 g. In addition, as shown in FIG. 21(b), when the daily change in sunlight during the day was simulated by adjusting the amount of light (PAR), it was confirmed that the transpiration rate (E) and the carbon dioxide assimilation rate (A) were linked to the PAR. Specifically, it was confirmed that the maximum value of $A_{NEW}$ decreased and the maximum value of $E_{NEW}$ increased three days after irrigation was stopped, that is, the influence of water stress was confirmed. Furthermore, 4 days after irrigation was stopped, $A_{NEW}$ was decreased in a wider range of PAR > 1000 $\mu$molm$^{-2}$s$^{-1}$, and $E_{NEW}$ was decreased in the low PAR range while maintaining the maximum value of $E_{rrEw}$. However, these effects were mitigated by resuming irrigation on the 4th day.

From the results of FIGS. 20 and 21, it was confirmed that the measurement according to the present invention can measure the time-dependent changes in the biological information of a plant easily and with high accuracy when a leaf of a plant is subjected to water stress.

From the above results, it has been confirmed that the biological information measuring device and biological information measuring method of the present invention can measure the biological information of a plant body easily and continuously, and can be suitably used as a biological information measuring device and a biological information measuring method.

DESCRIPTION OF REFERENCE CHARACTERS

1 Biological information measuring device
2a, 2b, 2c Container
3 Sensor
4 Wall
5 Permeable membrane
6a, 6b, 6c, 6d Space
7 Plant
8 Leaf
9 Stem
10 Gas barrier membrane
11 Film
12 sealing layer
13 Chamber

**Claims**

1. A biological information measuring device comprising a container for covering at least a part of a leaf of a plant, and a sensor for measuring the concentration of at least one of water vapor and carbon dioxide inside and outside the container,
   wherein at least a part of a wall constituting the container is constituted by a permeable membrane having permeation resistance to water vapor or carbon dioxide.

2. The biological information measuring device according to claim 1, further comprising a calculator for calculating at least one of the transpiration rate and the carbon dioxide assimilation rate in the leaf of the plant based on the concentration difference of at least one of water vapor and carbon dioxide between inside and outside the container and the permeation resistance.

3. A biological information measuring device comprising a permeable membrane having permeation resistance to water vapor or carbon dioxide; a sealing layer provided on the surface of the permeable membrane so as to surround a predetermined region of the surface, and for adhering a leaf of a plant to the permeable membrane; and a sensor provided inside and outside the predetermined region on the surface of the permeable membrane, and for measuring the concentration of at least one of water vapor and carbon dioxide.

4. The biological information measuring device according to claim 3, further comprising a calculator for calculating at least one of the transpiration rate and the carbon dioxide assimilation rate in the leaf of the plant based on the concentration difference of at least one of water vapor and carbon dioxide between inside and outside a space formed by the permeable membrane, the sealing layer, and the leaf of the plant in the predetermined region of the permeable membrane, and based on the permeation resistance.

5. The biological information measuring device according to any one of claims 1 to 4, wherein the permeable membrane has a plurality of pores.

6. A method for measuring biological information, comprising a step of covering at least a part of a leaf of a plant with a container at least a part of which is constituted by a permeable membrane having permeation resistance to water vapor or carbon dioxide to form a space between the container and the leaf, or storing a leaf of a plant within a space of the container, a step of measuring the concentration of at least one of water vapor and carbon dioxide inside and outside the space, and a step of calculating at least one of the transpiration rate and the carbon dioxide assimilation rate in the leaf of the plant based on the concentration difference measured between the inside and outside of the space of at least one of water vapor and carbon dioxide, and the permeation resistance.

7. The method for measuring biological information according to claim 6, comprising a step of further covering the back side of the leaf of the plant covered by the container with a gas barrier membrane, or a step of covering the back side of the leaf of the plant with another container at least a part of which is constituted by a permeable membrane having permeation resistance to water vapor or carbon dioxide, to further form a space between the other container and the back side of the leaf.

8. A method for measuring biological information, comprising a step of using a film constituted by a permeable membrane having permeation resistance to water vapor or carbon dioxide and a sealing layer provided on the surface of the permeable membrane so as to surround a predetermined region of the surface, to adhere at least a part of a leaf of a plant to the sealing layer of the film, thereby forming a space between the film and the leaf in the predetermined region of the film, a step of measuring the concentration of at least one of water vapor and carbon dioxide inside and outside the space, and a step of calculating at least one of the transpiration rate and the carbon dioxide assimilation rate in the leaf of the plant based on the concentration difference measured between the inside and outside of the space of at least one of water vapor and carbon dioxide, and the permeation resistance.

9. The method for measuring biological information according to claim 8, comprising a step of further covering the back side of the leaf of the plant covered by the film with a gas barrier membrane; and a step of using another film constituted by a permeable membrane having permeation resistance to water vapor or carbon dioxide and a sealing layer provided on the surface of the permeable membrane so as to surround a predetermined region of the surface, to adhere the back side of the leaf of the plant to the sealing layer of the other film, thereby further forming a space between the other film and the back side of the leaf in the predetermined region of the other film.

10. The method for measuring biological information according to any one of claims 6 to 9, wherein the permeable membrane has a plurality of pores.

[FIG. **1**]

[FIG. **2**]

[FIG. 3]

[FIG. 4]

[FIG. 5]

[FIG. 6]

[FIG. 7]

[FIG. **8**]

( a )

( b )

[FIG. 9]

（a）

（b）

[FIG. 10]

（a）

（b）

[FIG. 11]

(a)

(b)

[FIG. **12**]

[FIG. 13]

（a）

（b）

[FIG. 14]

grape

[FIG. 15]

（a）

sunflower

（b）

sunflower

[FIG. 16]

[FIG. 17]

[FIG. 18]

（a）

（b）

[FIG. 19]

[FIG. 20]

[FIG. 21]

( a )

number of days after stopping irrigation

( b )   number of days after stopping irrigation

[FIG. **22**]

[FIG. **23**]

[FIG. 24]

# EP 4 659 564 A1

| INTERNATIONAL SEARCH REPORT | International application No. |
| --- | --- |
| | **PCT/JP2024/001028** |

**A. CLASSIFICATION OF SUBJECT MATTER**

*A01G 7/00*(2006.01)i; *G01N 21/31*(2006.01)i
FI: A01G7/00 603; G01N21/31

According to International Patent Classification (IPC) or to both national classification and IPC

**B. FIELDS SEARCHED**

Minimum documentation searched (classification system followed by classification symbols)

A01G7/00; G01N21/31; G01N33/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2019-170247 A (EHIME UNIV) 10 October 2019 (2019-10-10)<br>entire text, all drawings | 1-10 |
| A | JP 5-14753 Y2 (KOITO KOGYO KK) 20 April 1993 (1993-04-20)<br>entire text, all drawings | 1-10 |
| A | JP 63-33669 B2 (SHIMADZU CORPORATION) 06 July 1988 (1988-07-06)<br>entire text, all drawings | 1-10 |
| A | US 4789436 A (THE UNITED STATES OF AMERICA AS REPRESENTED BY THE UNITED STATES DEPARTMENT OF ENERGY) 06 December 1988 (1988-12-06)<br>entire text, all drawings | 1-10 |

☐ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **21 March 2024** | **02 April 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/001028**

| Patent document cited in search report | | | Publication date (day/month/year) | Patent family member(s) | | | Publication date (day/month/year) |
|---|---|---|---|---|---|---|---|
| JP | 2019-170247 | A | 10 October 2019 | US entire text, all drawings WO | 2021/0007288 2019/188848 | A1 A1 | |
| JP | 5-14753 | Y2 | 20 April 1993 | (Family: none) | | | |
| JP | 63-33669 | B2 | 06 July 1988 | (Family: none) | | | |
| US | 4789436 | A | 06 December 1988 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019170247 A